# EUROPEAN PATENT APPLICATION

(11) **EP 3 936 238 A1**
(43) Date of publication of application: **12.01.2022**
(21) Application number: 20765757.8
(22) Date of filing: 03.03.2020
(51) Int. Cl.: B05D 1/36, C12M 3/00, C12N 5/07, C12N 5/077, C12N 1/00

(54) **CELL TISSUE PRODUCTION METHOD, CELL TISSUE PRODUCTION SET, AND CULTIVATION CONTAINER CONTAINING CELL TISSUE PRODUCED BY SAID PRODUCTION METHOD**

(30) Priority: 06.03.2019 JP 2019040785; 25.04.2019 JP 2019083571
(71) Applicant: Osaka University, Suita-shi, Osaka 565-0871 (JP); NTN Corporation, Osaka-shi, Osaka 550-0003 (JP)
(72) Inventor: AKASHI, Mitsuru, Suita-shi, Osaka 565-0871 (JP); AKAGI, Takami, Suita-shi, Osaka 565-0871 (JP); ODA, Atsushi, Iwata-shi, Shizuoka 438-8510 (JP); CHIKAE, Shohei, Iwata-shi, Shizuoka 438-8510 (JP)
(74) Representative: Hoffmann Eitle
(86) International application number: PCT/JP2020/009897
(87) International publication number: WO 2020/179929

(57) **Abstract**

This cellular tissue production method can produce a cellular tissue with high density and high shape retainability while the cell viability is maintained, and includes: a first application step of applying a first application liquid to an application target; and a second application step of applying and layering a second application liquid onto the first application liquid applied in the first application step, wherein the second application step includes subjecting an interface between the first application liquid and the second application liquid to gelation while the first application liquid is kept fluid.

## Description

### TECHNICAL FIELD

The present invention relates to a method for producing a cellular tissue, a cellular tissue production set suitable for the method, and cultureware containing a cellular tissue(s) produced by the production method.

### BACKGROUND ART

Technology for *ex vivo* cellular three-dimensional tissue construction is critical in research on drug discovery and regenerative medicine. Human biological tissue-resembling cellular tissue construction, in particular, has been sought.

In the case of *ex vivo* cell manipulation, it is common to culture cells two-dimensionally by using cultureware, such as a plastic or glass dish. However, cells actually proliferate in vivo three-dimensionally to form a tissue or organ. Thus, in order to realize in vivo resembling culture conditions, it is critical for progression in drug discovery research and regenerative medicine study to construct a three-dimensional cellular tissue and use the cellular tissue for evaluation and experiment.

Each cellular tissue (cell assembly) obtained by assembling or aggregating cells is placed on, for instance, each well (recessed portion) aligned on a well plate to evaluate each cellular tissue. This assay has been widely used in evaluation of cellular function and screening for a compound effective as a novel pharmaceutical. Such cellular tissues may be assayed to evaluate many items within a short time by using a small amount of sample. This is advantageous from the viewpoint of making the evaluation rapid, simple, safe, reproducible, and highly reliable.

Examples of technology for precisely patterning cells on a substrate include a method of processing a substrate using a photolithography technique for controlling cell adhesion and a method of directly printing cells for arrangement and immobilization. Recently, 3D printer technology for three-dimensionally (3D) arranging and layering cells has advanced and cell assembly construction using a 3D printer has been investigated. Here, it has considerably been developed to apply a tissue model obtained from cellular tissues to drug discovery research and regenerative medicine (see Patent Documents 1 to 8).

In recent years, printed electronics technology, in which a fine circuit such as an RFID tag is drawn by a printing (application) method, has been rapidly developed. Examples of a method for forming a micro-circuit pattern or an electrode pattern include an inkjet technique or a dispenser technique. Such printed electronics technology has been not only applied to the formation of circuits or electrodes, but also studied as bioprinting technology applicable to a cellular tissue preparation technique (see, for instance, Patent Document 9).

The present applicant has a technology using an application needle as one of printing technologies, and has conducted R&D of ex vivo three-dimensional cellular tissue construction technology by applying the application needle technology to a bioprinting technique (see Patent Document 10). A method for micro-application of a liquid material by using an application needle is disclosed in, for instance, Patent Document 11. The purpose of such an application unit is to correct defects of a micro-pattern. Accordingly, an application liquid with a widely varied viscosity may be used to conduct micro-application. During application operation, a single application needle is protruded from a through-hole formed at the bottom of an application liquid container that retains the application liquid. The application needle is used for application such that the application liquid attached to the tip is brought into contact with an application target

The above application needle is applied to an application process such that iPS-derived cardiomyocytes are dispersed in a fibrinogen solution, applied, and then immersed in a thrombin solution to immobilize a tissue by gelation. In the process, if fibrinogen is used as a gelatinizer at the time of applying cardiomyocytes, the application is performed by applying a first liquid, namely an application preparation obtained by dispersing cardiomyocytes and fibrinogen and by adding a second liquid, namely thrombin as a gelling initiator, to cover the application preparation. Unfortunately, prior to the application step, a phenomenon was observed where unintended gelation occurred inside the first liquid including cardiomyocytes and fibrinogen. This has caused problems that a cardiomyocyte mass gelled inside the application liquid container causes clogging in some cases or the gelled cardiomyocytes remain in the application liquid container and cannot be applied in some cases.

### PATENT DOCUMENT

[Patent Document 1] JP 2008-126459 A
[Patent Document 2] JP 2008-017798 A
[Patent Document 3] JP 2010-022251 A
[Patent Document 4] JP 2015-229148 A
[Patent Document 5] JP 2016-087822 A
[Patent Document 6] JP 2017-163931 A
[Patent Document 7] JP 2017-169560 A
[Patent Document 8] JP 2017-131144 A
[Patent Document 9] JP 2016-526910 A
[Patent Document 10] JP 4802027 B
[Patent Document 11] WO 2019/088224 A

### SUMMARY OF THE INVENTION

The purpose of the present invention is to provide a bioprinting technology so as not to cause problems such as gelation inside an application liquid container prior to application step execution and clogging accompanied by the gelation in the above bioprinting technique. Surprisingly, it has been found that a bioprinting technology capable of achieving the above goal can be used to produce a cellular tissue having high density and high shape retainability while cell viability is maintained.

The invention provides a method for producing a cellular tissue, capable of producing a cellular tissue having high density and high shape retainability while cell viability is maintained without causing problems such as gelation inside an application liquid container and clogging accompanied by the gelation.

In addition, the invention provides a method for producing a cellular tissue, capable of producing a cellular tissue having high density and high shape retainability while cell viability is maintained and producing a plurality of cellular tissues and a plurality of types of cellular tissues in one well (culture medium), and cultureware containing a cellular tissue(s) produced by the production method.

Specifically, the invention provides a method for producing a cellular tissue, including: a first application step of applying a first application liquid to an application target; and a second application step of applying and layering a second application liquid onto the first application liquid applied in the first application step. More specifically, the second application step includes subjecting an interface between the first application liquid and the second application liquid to gelation while the first application liquid is kept fluid.

According to the method of the invention, in the application step using bioprinting technology such as an application needle, problems such as gelation inside an application liquid container prior to the application step execution and clogging accompanied by the gelation do not occur.

In addition, the invention makes it possible to produce a cellular tissue having high density and high shape retainability while cell viability is maintained, and to produce a plurality of cellular tissues and a plurality of types of cellular tissues in one well (culture medium). This exerts a remarkable effect allowing for evaluation of many samples just by a single evaluation experiment.

### BRIEF DESCRIPTION OF DRAWINGS

Fig. 1 is an entire view showing a micro-applicator used in embodiment 1 according to the invention.
Fig. 2 is a diagram illustrating an application needle holder part mounted on an application unit in the micro-applicator.
Fig. 3 is a diagram showing tip portions of application needles provided to the application needle holder part.
Fig. 4 is diagrams schematically illustrating cell application operation in the application unit.
Fig. 5 is photographs showing images of liquid droplet spots observed under a phase-contrast microscope in application experiment 1.
Fig. 6 is photographs showing images of liquid droplet spots observed under a phase-contrast microscope in application experiment 2.
Fig. 7 is photographs showing images of liquid droplet spots observed under a phase-contrast microscope in application experiment 3.
Fig. 8 is photographs showing images of liquid droplet spots observed under a phase-contrast microscope in application experiment 4.
Fig. 9 is a graph showing the experimental results of application experiment 5.
Fig. 10 is an image picture showing a cell assembly produced in application experiment 6.
Fig. 11 is pictures showing viable cell/dead cell (Live/Dead) fluorescently stained images in application experiment 7.
Fig. 12 is a photograph showing images of states of myocardial tissue bodies, produced in application experiment 8, immediately after application.
Fig. 13 is a photograph showing images of states of the myocardial tissue bodies shown in Fig. 12 after 6 days of culturing.
Fig. 14 is a photograph showing an enlarged image of one of the myocardial tissue bodies after 5 days of culturing.
Fig. 15 is a graph showing a contraction-relaxation rate obtained by analyzing a pulsation video of the myocardial tissue body cultured for 5 days as shown in Fig. 14.
Fig. 16 is schematic diagrams each illustrating a well of multi-well cultureware in a method for producing a cellular tissue according to embodiment 2 of the invention while a cellular tissue(s) is produced on a flat-bottom well.
Fig. 17 is schematic plan views each illustrating an example in which cellular tissues are produced in a variously shaped well in the method for producing a cellular tissue of embodiment 2.
Fig. 18 is a schematic overview for explaining an application process using an application needle as a method for producing a cellular tissue according to embodiment 3 of the invention and sedimentation and precipitation of cells after application.
Fig. 19 is a view showing an example of a micro-applicator in embodiment 3.
Fig. 20 is a view showing an example of an applicator main body.
Fig. 21 is photographs showing the application results of how 40 wells of a 96-well plate looked after application.
Fig. 22 is a phase contrast micrograph of a cellular tissue.
Fig. 23 is an image showing the result of measuring the thickness of a cellular tissue by HE staining.
Fig. 24 is a phase contrast micrograph of a cellular tissue observed by fluorescent staining.
Fig. 25 is photographs showing formation of a gel-like material in which cardiomyocytes in an application liquid container are embedded.
Fig. 26 is a phase contrast micrograph of a cellular tissue obtained using a conventional method.
Fig. 27 is a schematic diagram illustrating each step in a method for producing a cellular tissue according to embodiment 4 of the invention.
Fig. 28 is a time chart for the first application step, the second application step, and an in-medium formation step in the method for producing a cellular tissue of embodiment 4.
Fig. 29 is a photograph showing an image of a three-dimensional cellular tissue in the case of dispersing cardiomyocytes and thrombin in a first application solution.
Fig. 30 is a photograph showing an image of the result of measuring the thickness of a produced cellular tissue by fluorescent staining.
Fig. 31 is a photograph showing an image of the result of observing a produced cellular tissue by fluorescent staining.
Fig. 32 is a front view showing a micro-applicator used in a method for producing a cellular tissue according to embodiment 5 of the invention.
Fig. 33 is a schematic plan view showing an example in which a micro-applicator was used to produce a plurality of cellular tissues in cultureware.
Fig. 34 is a flowchart for producing a plurality of cellular tissues shown in Fig. 33.
Fig. 35 is a schematic plan view showing a modification example in which a micro-applicator was used to produce a plurality of cellular tissues in cultureware.
Fig. 36 is a flowchart for producing a plurality of cellular tissues shown in Fig. 35.

### EMBODIMENT(S) FOR CARRYING OUT THE INVENTION

First, various items will be described, including a method for producing a cellular tissue according to the invention and a cellular tissue prepared using cultureware containing a cellular tissue produced by the production method.

Note that as used herein, the cellular tissue refers to a cell assembly in a state in which cells are assembled, aggregated, and/or layered to form a functional tissue.

A method for producing a cellular tissue according to item 1 of the invention includes: a first application step of applying a first application liquid to an application target; and a second application step of applying and layering a second application liquid onto the first application liquid applied in the first application step and subjecting an interface between the first application liquid and the second application liquid to gelation.

In a method for producing a cellular tissue according to item 2 of the invention, the second application step in item 1 may include subjecting an interface between the first application liquid and the second application liquid to gelation while the first application liquid is kept fluid.

In a method for producing a cellular tissue according to item 3 of the invention, the first application liquid in item 1 or 2 may contain a cell(s) and a gelling initiator.

In a method for producing a cellular tissue according to item 4 of the invention, the second application liquid in any one of items 1 to 3 may contain a gelatinizer.

A method for producing a cellular tissue according to item 5 of the invention may include: a first application step of applying a first application liquid containing a cell(s) and a gelling initiator to an application target; and a second application step of applying and layering a second application liquid containing a gelatinizer onto the first application liquid applied in the first application step, and starting a gelation reaction at an interface between the first application liquid and the second application liquid.

A method for producing a cellular tissue according to item 6 of the invention may include: a first application step of applying a first application liquid containing a cell(s) to an application target; and a second application step of applying and layering a second application liquid containing collagen, gelatin and/or agar as a gelatinizer onto the first application liquid applied in the first application step, and starting a gelation reaction by heating or cooling at an interface between the first application liquid and the second application liquid.

A method for producing a cellular tissue according to item 7 of the invention may include: a first application step of applying a first application liquid containing a cell(s) to an application target; and a second application step of applying and layering a second application liquid containing a photocrosslinkable gelatin hydrogelatinizer onto the first application liquid applied in the first application step, and starting a gelation reaction by light irradiation at an interface between the first application liquid and the second application liquid.

A method for producing a cellular tissue according to item 8 of the invention may include a step of immersing the application target, to which the first and second application liquids have been applied in any one of items 1 to 7, in a medium and culturing the cell(s).

In a method for producing a cellular tissue according to item 9 of the invention, the first application liquid in any one of items 1 to 8 may contain a thickening polysaccharide.

In a method for producing a cellular tissue according to item 10 of the invention, the second application liquid in any one of items 1 to 9 may contain a thickening polysaccharide.

In a method for producing a cellular tissue according to item 11 of the invention, the gelling initiator in any one of items 3 to 5 is thrombin.

In a method for producing a cellular tissue according to item 12 of the invention, the gelatinizer in any one of item 4 or 5 may be fibrinogen, collagen, gelatin, or a mixture of two or more thereof.

In a method for producing a cellular tissue according to item 13 of the invention, the thickening polysaccharide in any one of item 9 or 10 may be sodium hyaluronate, sodium alginate, or a mixture thereof.

In a method for producing a cellular tissue according to item 14 of the invention, the cell(s) in any one of items 3 to 13 may be a cardiomyocyte(s).

A cellular tissue formation set according to item 15 of the invention includes at least a first container containing a first application liquid containing a cell(s) and a gelling initiator and a second container containing a second application liquid containing a gelatinizer.

In a cellular tissue formation set according to item 16 of the invention, the first application liquid in item 15 may contain a thickening polysaccharide.

In a cellular tissue formation set according to item 17 of the invention, the second application liquid in item 15 or 16 may contain a thickening polysaccharide.

In a cellular tissue formation set according to item 18 of the invention, the gelling initiator in any one of items 15 to 17 may be thrombin.

In a cellular tissue formation set according to item 19 of the invention, the gelatinizer in any one of items 15 to 18 may be fibrinogen, collagen, gelatin, or a mixture of two or more thereof.

In a cellular tissue formation set according to item 20 of the invention, the thickening polysaccharide in any one of items 16 to 19 may be sodium hyaluronate, sodium alginate, or a mixture thereof.

In a cellular tissue formation set according to item 21 of the invention, the cell(s) in any one of items 15 to 20 may be a cardiomyocyte(s).

A cellular tissue according to item 22 of the invention is a cellular tissue produced by the method for producing a cellular tissue according to any one of items 1 to 14.

In a method for producing a cellular tissue according to item 23 of the invention, the first application step in any one of items 1 to 14 is carried out by contact application using an application needle.

In a method for producing a cellular tissue according to item 24 of the invention, application operations of the first and second application steps in any one of items 1 to 14 or 23 are executed at a plurality of application target positions on an identical well and an in-medium formation step is executed.

In a method for producing a cellular tissue according to item 25 of the invention, the application operations of the first and second application steps in item 24 may be executed at the plurality of application target positions on the identical well, and the in-medium formation step may then be executed.

In a method for producing a cellular tissue according to item 26 of the invention, the application operations of the first and second application steps in item 24 may be executed at the plurality of application target positions on the identical well, and then the in-medium formation step may be executed at the application target positions.

In a method for producing a cellular tissue according to item 27 of the invention, the application operation of the first step may be executed at the plurality of application target positions on the identical well, and then the application operation of the second application step may be executed at the plurality of application target positions on the identical well, and then the in-medium formation step is executed at the plurality of application target positions on the identical well in item 24.

In a method for producing a cellular tissue according to item 28 of the invention, the in-medium formation step includes applying a third application liquid so that the second application liquid including the first application liquid is immersed in the third application liquid in any one of items 24 to 27.

In a method for producing a cellular tissue according to item 29 of the invention, a cellular tissue containing the same type of cell(s) may be produced at the plurality of application target positions on the identical well in any one of items 24 to 28.

In a method for producing a cellular tissue according to item 30 of the invention, a cellular tissue containing a different type of cell(s) may be produced at the plurality of application target positions on the identical well in any one of items 24 to 28.

In a method for producing a cellular tissue according to item 31 of the invention, an inkjet technique or a dispenser technique may be used for the second application step of applying and layering the second application liquid onto the first application liquid in any one of items 24 to 30.

In a method for producing a cellular tissue according to item 32 of the invention, the application liquids used during cellular tissue formation in any one of items 24 to 31 may be a two-component gelation solution.

Cultureware according to item 33 of the invention includes a plurality of cellular tissues on an identical well.

Cultureware according to item 34 of the invention may include a plurality of the same type of cellular tissues on the identical well in item 33.

Cultureware according to item 35 of the invention may include a plurality of different types of cellular tissues on the identical well in item 33.

Cultureware according to item 36 of the invention includes a plurality of wells, each well including a plurality of cellular tissues in any one of items 33 to 35.

In addition, a method for producing a cellular tissue according to one embodiment of the invention may include: a first application step of applying a first application liquid to an application target; a second application step of applying and layering a second application liquid onto the first application liquid applied in the first application step; and an in-medium formation step of applying a third application liquid so that the second application liquid including the first application liquid is immersed in the third application liquid, wherein application operations of the first and second application steps are executed at a plurality of application target positions on an identical well and the in-medium formation step is executed.

The following method for producing a cellular tissue will be specifically exemplified using the below-described embodiments. A micro-applicator may be used to apply, per application in a very short time, several pL (picoliter) of liquid micro-droplet attached to the tip of an application needle, for instance, to apply, in 0.1 sec, the liquid micro-droplet onto a given target position with high precision. This micro-applicator has high safety and reproducibility, and can be automated to produce a cellular tissue (cell assembly) in a short time at a large scale in a highly reliable manner.

In the invention, a high-speed micro-applicator may be used to apply a highly viscous material that cannot be handled by conventional printers. For instance, even a highly viscous solution containing cells and a gelatinizer may be applied reliably at given positions in a short time to produce a plurality of cellular tissues (cell assemblies) with high precision. As a result, the invention makes it possible to optionally control and arrange desired cells two-dimensionally and three-dimensionally. This process may be automated to produce various cellular tissues in large quantity under aseptic conditions. Thus, the method for producing a cellular tissue according to the invention has high safety and reproducibility, and makes it possible to automatically produce a large amount of reliable cellular tissue.

Next, with reference to the accompanying drawings attached, a method for producing a cellular tissue and a cellular tissue prepared by the production method will be described using embodiments. Note that the methods for producing a cellular tissue according to the invention are not limited to configurations using micro-applicators in the below-described embodiments. The methods can be implemented by cell application operation (cell application process) in the technical ideas comparable to those for the cell application operation (cell application process) carried out in micro-applicators.

### (Embodiment 1)

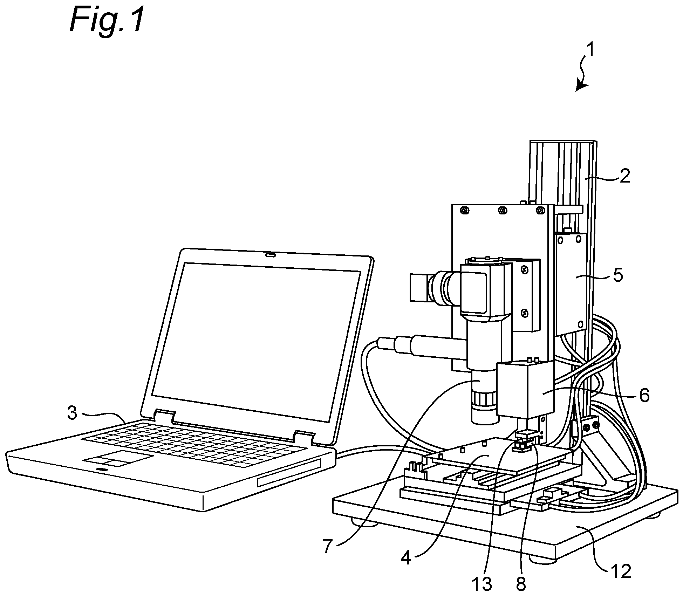

Hereinafter, embodiment 1, which relates to a method for producing a cellular tissue according to the invention and a cellular tissue prepared by the production method, will be specifically described with reference to the accompanying drawings. Fig. 1 is an entire view illustrating the micro-applicator 1 used in embodiment 1. As shown in Fig. 1, the micro-applicator 1 is provided with an applicator main body 2 and a display/control unit 3 configured to display, control, and set the applicator main body 2. The display/control unit 3 of the micro-applicator 1 in embodiment 1 includes, as a component, what is called a personal computer (PC).

The applicator main body 2 of the micro-applicator 1 includes: an XY table 4 movable over a main body base 12 in a horizontal direction; a Z table 5 movable in a top-to-bottom direction (vertical direction) with respect to the XY table 4; an application unit 6 fixed to a driving mechanism movable, like the Z table 5, in the top-to-bottom direction; and an optical detection unit (e.g., a CCD camera) 7 configured to observe an application target on the XY table 4. For instance, a substrate, on which an application liquid 10, a cell-containing solution, is applied to form a plurality of cellular tissues, is placed on and fixed to the XY table 4.

The application unit 6 in the micro-applicator 1 as structured above is configured to carry out cell application operation for aligning and forming a plurality of cellular tissues on, for instance, a substrate over the XY table 4. Hereinbelow, the structure of the application unit 6 and the cell application operation by using the application unit 6 will be described.

### [Structure of Application Unit]

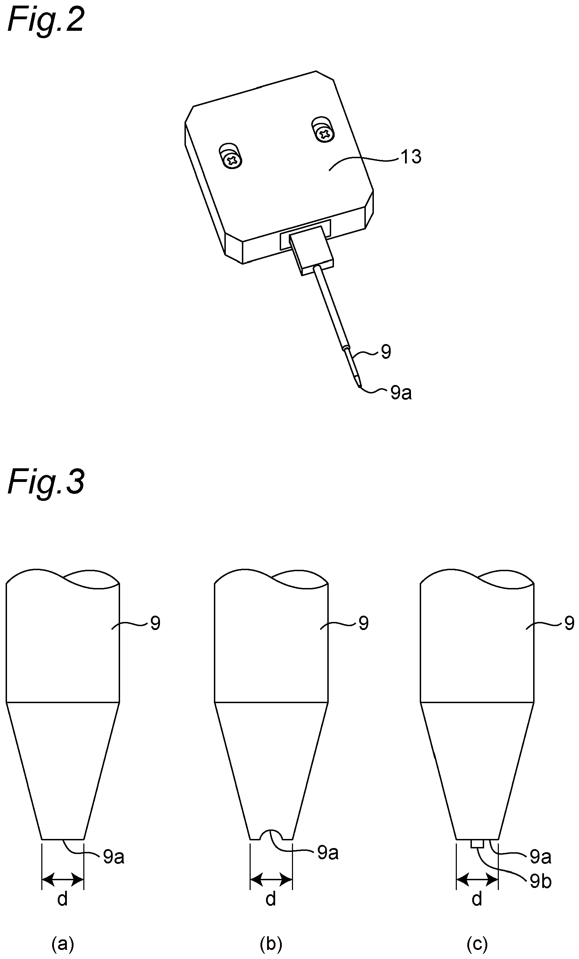

Fig. 2 is a diagram illustrating an application needle holder part 13 mounted on the application unit 6. An application needle 9 protrudes from the application needle holder part 13. Fig. 3 is diagrams illustrating a tip portion of each application needle 9. Regarding the application needle 9 in this embodiment, a tip 9a of cone-shaped leading edge portion has a flat surface (is flat) so as to face a horizontal surface of the XY table 4 (see (a) of Fig. 3). That is, the flat surface of the tip 9a is a flat surface perpendicular to the vertical direction. The diameter d of the tip 9a greatly contributes to the shape of the cellular tissue to be produced as described later. In embodiment 1, the tip 9a of the application needle 9 has a diameter d of from 50 to 330 µm. In embodiment 1, as shown in (a) of Fig. 3, the leading edge portion of the application needle 9 is shaped like a cone. The tip 9a has a horizontal surface. Accordingly, the tip 9a may be polished to a horizontal surface so as to easily adjust the diameter d of the tip 9a to a desired value ranging from, for instance, 50 to 330 µm.

Note that, in embodiment 1, the tip 9a of the application needle 9 is explained using an example of horizontal flat surface configuration (see (a) of Fig. 3). This tip 9a surface shape may have a recessed surface (semi-spherical surface) with a prescribed diameter, such as a diameter of 30 µm or less, and cells may be retained on the recessed surface so as to carry out cell application operation (see (b) of Fig. 3). Such formation of the tip 9a of the application needle 9 into a recessed surface makes it possible to produce a cellular tissue having a desired cell density and cell arrangement by the cell application operation using the application needle 9. In addition, as shown in (c) of Fig. 3, the tip 9a of the application needle 9 may have a stepped protrusion 9b. This configuration with such a protrusion 9b makes it possible to produce a cellular tissue extending upward relative to a substrate by performing continuous and repeated application over the substrate while a needle tip is shifted upward by a given distance, such as 0.5 µm, every cell application operation.

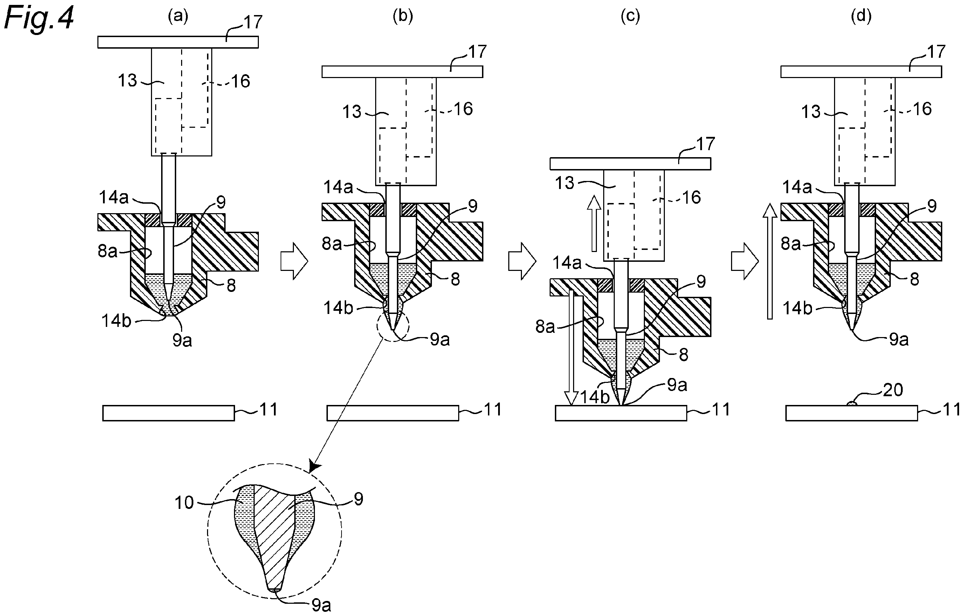

Fig. 4 is diagrams schematically illustrating cell application operation in the application unit 6. As shown in Fig. 4, the application unit 6 includes: an application liquid container 8 having an application liquid reservoir 8a in which a prescribed amount of application liquid 10, a cell-containing solution, is stored; and the application needle holder part 13 equipped with the application needle 9 that penetrates through the application liquid reservoir 8a. The application needle holder part 13 is provided with a sliding mechanism part 16 that slidably holds the application needle 9 in the top-to-bottom direction (vertical direction). The application needle holder part 13 is detachably provided at a given position on a driving mechanism part 17, and, for instance, may be detachable from the driving mechanism part 17 by using magnetic force of a magnet.

The application needle holder part 13 is so fixed to the driving mechanism part 17 in the applicator main body 2 and is configured to reciprocally move between prescribed distances at a high speed in the top-to-bottom direction (vertical direction). The display/control unit 3 executes, for instance, settings of driving control of such a driving mechanism part 17 and driving control of the YX table 4 and the Z table 5. The sliding mechanism part 16 provided in the application needle holder part 13 holds the application needle 9 so as to be able to reciprocate vertically. The sliding mechanism part 16 is configured to bring the application liquid 10 held by the tip 9a of the application needle 9 into contact with the application target and then reciprocate upward. Note that the sliding mechanism part 16 is configured such that the tip 9a of the application needle 9 may descend to come into contact with, for instance, the substrate 11; at this time, the application needle 9 slides along the sliding mechanism part 16 in a vertical direction to be lifted from the contact position.

As described above, the sliding mechanism part 16 of the application unit 6 in embodiment 1 is configured such that the application liquid 10 held by the tip 9a of the application needle 9 is subject to contact application on the application target. The tip 9a of the application needle 9 reciprocates upward and downward while the application target contact position is a return point. Note that the vertical reciprocating operation of the application needle 9 at that time is at a high speed. For instance, one reciprocating operation is set to be in preferably 0.5 sec or less and more preferably 0.1 sec or less.

As described above, the application needle holder part 13 in the application unit 6 is provided with the vertically slidable sliding mechanism part 16 while holding the application needle 9 and is detachably fixed to the vertically moving driving mechanism part 17. In addition, the application needle holder part 13 is configured such that the application needle 9 can move in the top-to-bottom direction (vertical direction) and penetrate through the application liquid reservoir 8a that stores the application liquid 10, a cell-containing solution. An upper portion and a lower portion of the application liquid container 8 each have a hole (upper hole 14a or lower hole 14b) through which the application needle 9 penetrates.

### [Cell Application Operation]

Next, the cell application operation in the application unit 6, as schematically shown in Fig. 4, will be described. During the cell application operation illustrated in Fig. 4, the application needle 9 passes through the application liquid reservoir 8a of the application liquid container 8 and comes into contact with the substrate 11, which is an application target; and the cell-containing application liquid 10 is applied on the substrate 11 to form a liquid droplet spot. This cell application operation is repeated prescribed times and the application liquid 10 is applied multiple times to produce a desired cellular tissue(s) on the substrate 11.

(a) of Fig. 4 shows a waiting state during the cell application operation. In this waiting state, the application needle 9 is inserted from the upper hole 14a and the tip 9a of the application needle 9 is dipped into the application liquid 10 in the application liquid reservoir 8a. In such a waiting state (waiting step), the tip 9a of the application needle 9 is dipped into the application liquid 10, so that the application liquid 10 attached to the tip 9a is not dried. At that time, because the diameter of the lower hole 14b at the application liquid container 8 is very small (e.g., 1 mm or less), no leak of the application liquid 10 from the application liquid reservoir 8a occurs.

(b) of Fig. 4 shows a state (descending step) in which the tip 9a of the application needle 9 is projected from the lower hole 14b at the application liquid container 8 and the tip 9a descends toward the substrate 11 from the application liquid reservoir 8a. That is, (b) of Fig. 4 shows a descending state in which the tip 9a of the application needle 9 penetrates through and protrudes from the application liquid reservoir 8a of the application liquid container 8. During this descending state, the application liquid 10 is attached to the application needle 9 and the surface tension of the application liquid 10 attached causes the tip 9a of the application needle 9 to retain a certain volume of application liquid 10.

(c) of Fig. 4 shows a state (application step) in which the application liquid 10 held by the tip 9a of the application needle 9 comes into contact with a surface of the substrate 11 and the application liquid 10 is applied on the surface of the substrate 11. A liquid droplet spot of the application liquid 10 applied at that time corresponds to a certain volume of application liquid 10 held on the tip 9a of the application needle 9. Embodiment 1 is configured such that the application liquid 10 held on the tip 9a of the application needle 9 is brought into contact with the application target and is subject to application (contact application). Embodiment 1 is also configured such that an impact load at the moment when the tip 9a of the application needle 9 is in contact with (comes into contact with) a surface of the substrate 11 is about 0.06 N or lower. In embodiment 1, as described above, because the application needle 9 is slidably held by the sliding mechanism part 16 so as to absorb a vertical shock, the impact load at the time of contact is a very small value.

(d) of Fig. 4 shows a state immediately after the application needle 9 is used to apply the application liquid 10 on a surface of the substrate 11 and shows a state in which the application needle 9 is being lifted. This lifting state is followed by transition into a waiting state in which the tip 9a of the application needle 9 is dipped into the application liquid 10 of the application liquid reservoir 8a (holding step).

As described above, the one cycle of cell application operation includes, in sequence, (a), (b), (c), (d), and (a) operations as illustrated in Fig. 4. In embodiment 1, the one cycle of cell application operation is conducted in 0.1 sec, and the cell application operation is executed in a short period. Note that in embodiment 1, the cell application operation is repeated prescribed times (e.g., 10 cycles) to produce a desired cellular tissue(s).

During the cell application operation using the micro-applicator 1 in embodiment 1 according to the invention, a very small volume of application liquid 10 attached to the tip of the application needle 9 is brought into contact with an application target; and a liquid droplet spot in an application volume of several pL (picoliter) can be applied and formed with high positional precision, such as positional precision of ±15 µm or less and preferably ±3 µm or less. In addition, it is possible to apply a material with a viscosity of the application liquid 10 of 1 × 10⁵ mPa·s or lower. This allows for application of highly viscous cell dispersion. During the cell application operation using the micro-applicator 1 in embodiment 1, it is possible to use, as an application material, a material with a viscosity of from 10 mPa·s to 1 × 10⁵ mPa·s (inclusive), which material has not been successfully used because nozzle-type printers such as inkjet printers have a problem of clogging, etc. In addition, a very small volume of application liquid 10 attached to the tip of the application needle 9 is brought into contact with an application target for application. Accordingly, the application is not affected by a variation in the vertical position of the application needle 9 and can be repeated using a desired application volume of application liquid 10 in a stable fashion. As such, in embodiment 1, a highly viscous cell dispersion can be precisely applied at a predetermined position on a surface of, for instance, the substrate 11. This makes it possible to produce a cellular tissue with a given pattern and three-dimensionally arranged cells. In view of the above, the method for producing a cellular tissue according to the invention exerts advantageous effects on progress in respective fields while the produced cellular tissue(s) is utilized in the fields of regenerative medicine and drug discovery research such as drug efficacy or safety evaluation screening.

### (Experimental Example 1)

The micro-applicator 1, which has been described in the above embodiment 1, was used to conduct application experiment 1 using application liquids 10 with different concentrations and viscosities. In this application experiment 1, 3 different application liquids 10, including 5%, 10%, and 20% gelatin PBS (phosphate buffer solution) solutions, were used to examine the shape of each liquid droplet spot.

Three application liquid containers 8 in the application unit 6 were provided, and gelatin was dissolved at 5, 10, or 20% weight by volume (%w/v) in phosphate buffer solution (PBS) to prepare, as the application liquids 10, three different gelatin PBS solutions. Note that each application liquid 10 used in this application experiment 1 is free of cells.

In application experiment 1, each application liquid container 8 was filled with 20 µL of 5%, 10%, or 20% gelatin PBS solution. A needle having a tip 9a (flat surface shape) with a diameter d of 100 µm was used as the application needle 9 in the application unit 6. In this application experiment 1, each application liquid 10 was subjected to point contact and was applied as 5 × 5 spots with a 150-µm interval on a slide glass fixed to the XY table 4. The liquid droplet spots formed on the slide glass by the application were observed under a phase-contrast microscope.

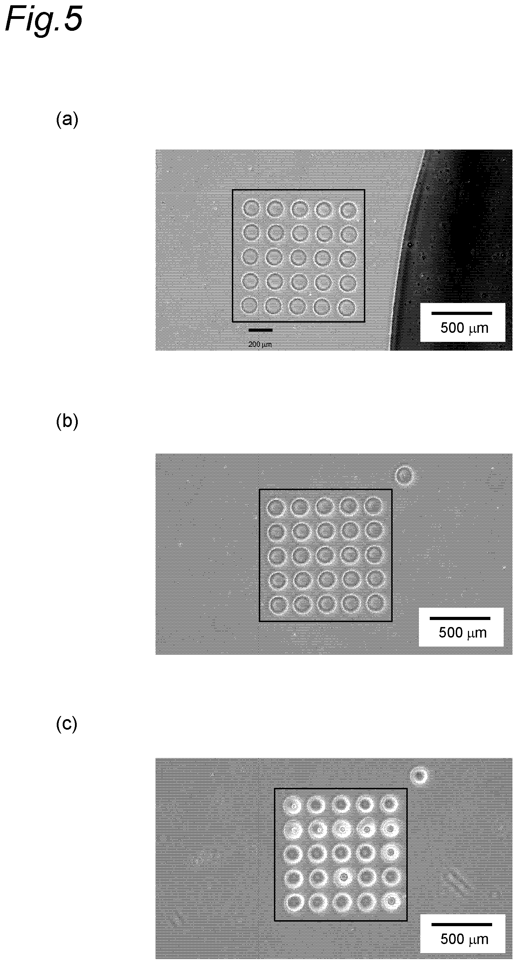

Fig. 5 is photographs showing images of the liquid droplet spots observed under a phase-contrast microscope in application experiment 1. (a) of Fig. 5 is an image showing liquid droplet spots formed by applying, as the application liquid 10, 5% gelatin PBS solution (with a viscosity of 3 mPa·s) on a slide glass by using an application needle 9 having a tip 9a with a diameter d (tip diameter) of 100 µm. (b) of Fig. 5 is an image showing liquid droplet spots formed by applying, as the application liquid 10, 10% gelatin PBS solution (with a viscosity of 30 mPa·s) on a slide glass by using the application needle 9 (with a tip diameter of 100 µm). In addition, (c) of Fig. 5 is an image showing liquid droplet spots formed by applying, as the application liquid 10, 20% gelatin PBS solution (with a viscosity of 220 mPa·s) on a slide glass by using the application needle 9 (with a tip diameter of 100 µm).

(a), (b), and (c) of Fig. 5 clearly demonstrate that when the application unit 6 of the micro-applicator 1 was used, the liquid droplet spots formed using the application liquids 10 even with different concentrations and viscosities had substantially the same shape (the 5% gelatin liquid droplet spots had a diameter of 136 ± 3 µm, the 10% gelatin liquid droplet spots had a diameter of 147 ± 1 µm, and the 20% gelatin liquid droplet spots had a diameter of 151 ± 1 µm). That is, application experiment 1 successfully verified constantly stable liquid droplet spots, which did not significantly depend on the gelatin concentration and viscosity. According to the experiment conducted by the present inventors, the diameter of each liquid droplet spot was within a size 1.3 to 1.6 times the tip diameter of the application needle 9, and the liquid droplet was not at least twice as large as the tip diameter. In another experimental example, an application liquid used in the application test is further highly viscous one, and is applied to an area about 1.0 to 1.2 times the diameter of the tip 9a of the application needle 9. Furthermore, in still another experimental example, the application test is performed using an application liquid having a low viscosity such that a liquid droplet becomes twice or more as large as the diameter of the tip 9a of the application needle 9 within a range in which the cellular tissues do not overlap in the well.

### (Experimental Example 2)

The micro-applicator 1, which has been described in the above embodiment, was used to conduct application experiment 2 using application needles 9 with different tip 9a diameters (tip diameters). In this application experiment 2, 5% gelatin PBS solution was used as the application liquid 10 to examine the shape of each liquid droplet spot formed. The application liquid 10 used in this application experiment 2 is free of cells.

In application experiment 2, three different application needles 9 having a tip diameter of 50 µm, 100 µm, or 150 µm were used. In application experiment 2, the application liquid 10 was subjected to point contact and was applied as 5 × 5 spots with a 150-µm interval on a slide glass fixed to the XY table 4. The liquid droplet spots formed on the slide glass by the application were observed under a phase-contrast microscope.

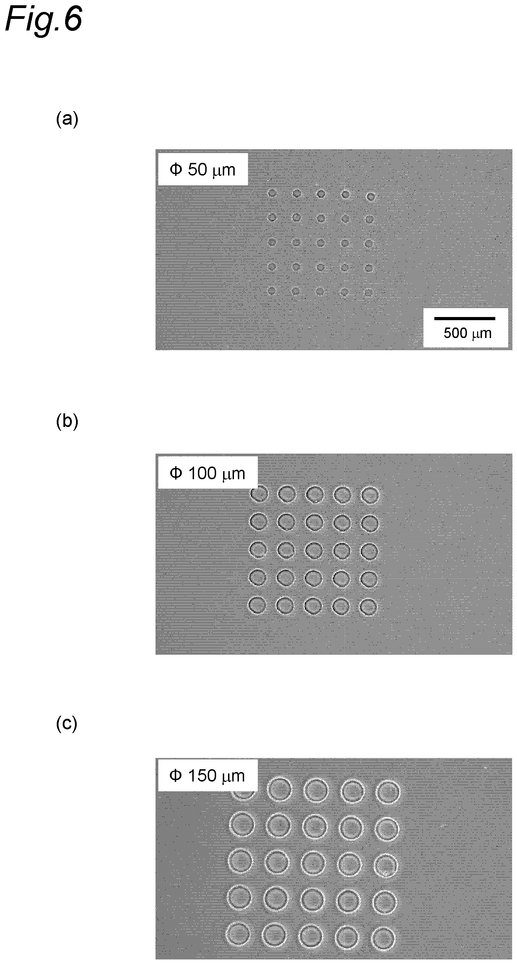

Fig. 6 is photographs showing images of the liquid droplet spots observed under a phase-contrast microscope in application experiment 2. (a) of Fig. 6 is an image showing liquid droplet spots formed by applying the application liquid 10 of 5% gelatin PBS solution on a slide glass by using an application needle 9 having a tip diameter of 50 µm. (b) of Fig. 6 is an image showing liquid droplet spots formed by applying the application liquid 10 of 5% gelatin PBS solution on a slide glass by using an application needle 9 having a tip diameter of 100 µm. (c) of Fig. 6 is an image showing liquid droplet spots formed by applying the application liquid 10 of 5% gelatin PBS solution on a slide glass by using an application needle 9 having a tip diameter of 150 µm.

(a), (b), and (c) of Fig. 6 successfully demonstrated that the liquid droplet spots formed had liquid droplet spot diameters approximately proportional to the diameters (50 µm, 100 µm, and 150 µm) of the application needles 10 (the 50-µm application needle: a liquid droplet spot diameter of 75 ± 2 µm; the 100-µm application needle: a liquid droplet spot diameter of 137 ± 3 µm; and the 150-µm application needle: a liquid droplet spot diameter of 219 ± 5 µm).

### (Experimental Example 3)

In application experiment 3, normal human dermal fibroblasts (NHDF) were dispersed at a concentration of 2 × 10⁷ cells/mL in 10% gelatin PBS solution to prepare an application liquid 10. In application experiment 3, the micro-applicator 1 was used to apply the application liquid 10 on a slide glass by contact application using three different application needles 9 with a tip diameter (tip diameter) of 100 µm, 150 µm, or 200 µm. The shapes of liquid droplet spots formed on the slide glass by the application were observed under a phase-contrast microscope.

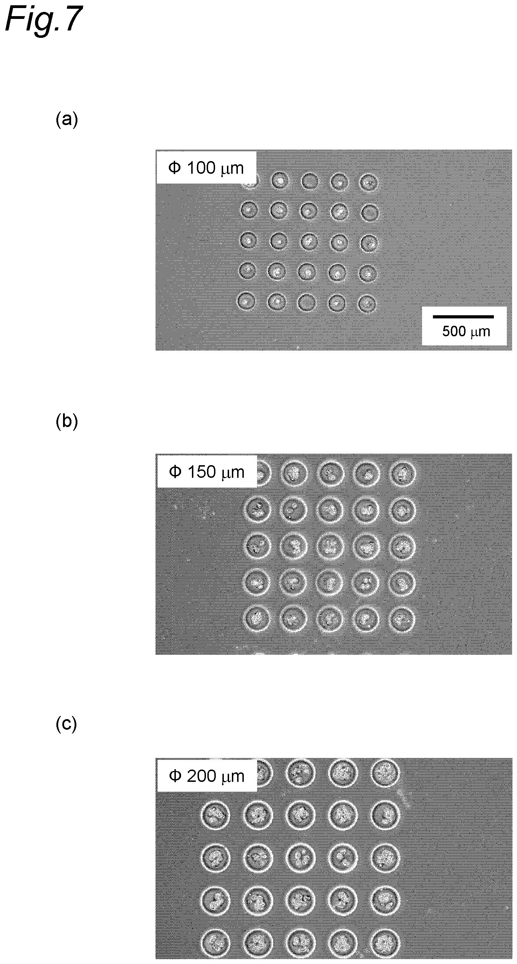

Fig. 7 is photographs showing images of the liquid droplet spots observed under a phase-contrast microscope in application experiment 3. (a) of Fig. 7 is an image showing liquid droplet spots formed by applying the NHDF-dispersed 10% gelatin PBS solution on a slide glass by using an application needle 9 having a tip diameter of 100 µm. (b) of Fig. 7 is an image showing liquid droplet spots formed by applying the NHDF-dispersed 10% gelatin PBS solution on a slide glass by using an application needle 9 having a tip diameter of 150 µm. (c) of Fig. 7 is an image showing liquid droplet spots formed by applying the NHDF-dispersed 10% gelatin PBS solution on a slide glass by using an application needle 9 having a tip diameter of 200 µm.

According to application experiment 3, when the application needle 9 having a tip diameter of 100 µm was used, one liquid droplet spot was found to contain 0 to 3 cells applied. When the application needle 9 having a tip diameter of 150 µm was used, one liquid droplet spot was found to contain 2 to 6 cells applied. In addition, one liquid droplet spot formed using the application needle 9 having a tip diameter of 200 µm was found to contain up to about 10 cells applied. Hence, by selecting the tip diameter of the application needle 9, it was found to be possible to control the number of cells applied in each liquid droplet spot to within about 1 to 10.

### (Experimental Example 4)

In application experiment 4, instead of normal human dermal fibroblasts (NHDF) in the above application experiment 3, a liver cancer cell line (HepG2) was used to likewise conduct an experiment. In application experiment 4, HepG2 was dispersed at a concentration of 5 × 10⁷ cells/mL in 10% gelatin PBS solution to prepare an application liquid 10.

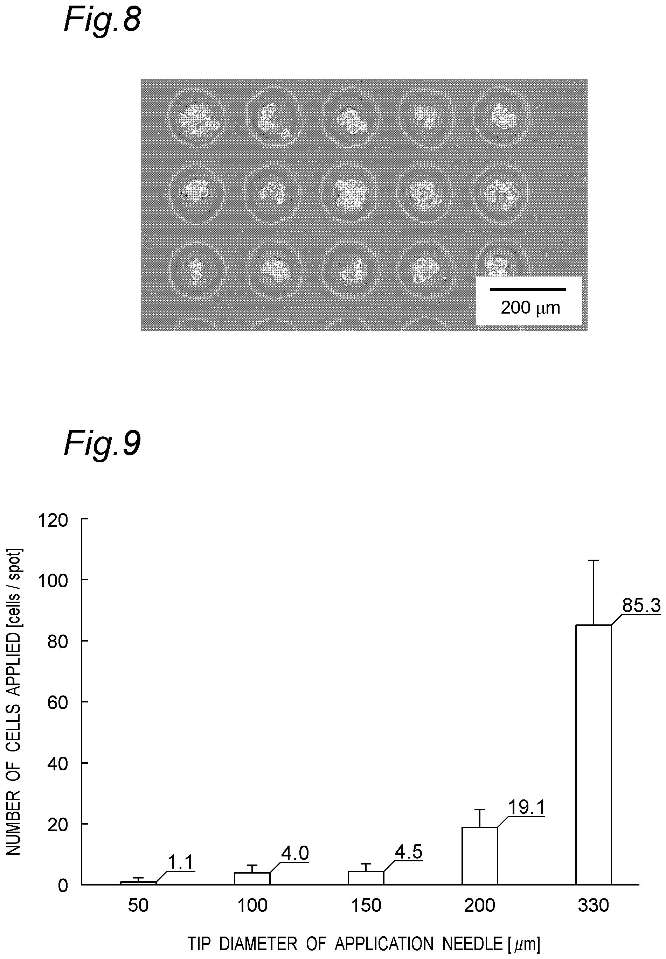

Fig. 8 is an image showing liquid droplet spots formed by applying the HepG2-dispersed 10% gelatin PBS solution on a slide glass by using an application needle 9 having a tip diameter of 100 µm. Even application experiment 4 demonstrated that a given number of cells were present in each liquid droplet spot, so that stable application was possible regardless of the kind of cells. In the below-described application experiment 5, how the tip diameter of the application needle 9 correlated to the number of cells applied and contained in each liquid droplet spot formed was investigated.

### (Experimental Example 5)

In application experiment 5, iPS cell-derived cardiomyocytes (iPS-CM) were dispersed at a concentration of 4 × 10⁷ cells/mL in PBS solution to prepare an application liquid 10 and respective application needles 9 with a tip diameter of 50 µm, 100 µm, 150 µm, 200 µm, or 330 µm were used to form liquid droplet spots. In application experiment 5, how the tip diameter of each application needle 9 correlated to the number of cells applied and contained in each liquid droplet spot formed was investigated.

In application experiment 5, the above application liquid 10 was applied once on a slide glass, and the number of cells after the application was calculated by fluorescence microscopy (using cells, the nuclei of which were stained with a fluorescent dye DAPI) and phase-contrast microscopy. In this application experiment 5, 20 or more liquid droplet spots formed by each of the application needles 9 with a tip diameter of 50 µm, 100 µm, 150 µm, 200 µm, or 330 µm were measured and averaged.

Fig. 9 is a graph showing the experimental results of application experiment 5. In Fig. 9, the ordinate represents the number of cells applied [cells/spot], and the abscissa represents the tip diameter of application needle 9 [µm]. As shown in Fig. 9, when iPS-CM was dispersed at a concentration of 4 × 10⁷ cells/mL in PBS solution, average 1.1 cells applied were present in each liquid droplet spot applied by using the application needle 9 with a tip diameter of 50 µm. Average 4.0 cells applied were present in each liquid droplet spot obtained by using the application needle 9 with a tip diameter of 100 µm; average 4.5 cells applied were present in each liquid droplet spot obtained by using the application needle 9 with a tip diameter of 150 µm; average 19.1 cells applied were present in each liquid droplet spot obtained by using the application needle 9 with a tip diameter of 200 µm; and average 85.3 cells applied were present in each liquid droplet spot obtained by using the application needle 9 with a tip diameter of 330 µm. Note that in Fig. 9, the error bars indicate the standard deviations (in the positive direction) of the number of cells applied using the respective application needles 9.

As described above, the tip diameter of each application needle 9 used was correlated with the number of cells applied and present in each liquid droplet spot formed, indicating that as the tip diameter of the application needle 9 became larger, the number of cells applied and present in each liquid droplet spot increased. Hence, by selecting the tip diameter of the application needle 9, it was successfully verified that the number of cells applied in each liquid droplet spot was able to be controlled to within a certain range.

### (Experimental Example 6)

In application experiment 6, the micro-applicator 1 was used to produce a cell assembly 20. In application experiment 6, normal human dermal fibroblasts (NHDF) were dispersed at a concentration of 2 × 10⁷ cells/mL in 2.5% sodium alginate PBS solution to prepare an application liquid 10. This application liquid 10 was charged into the application liquid container 8 of the application unit 6, and cell application operation was then executed by the micro-applicator 1.

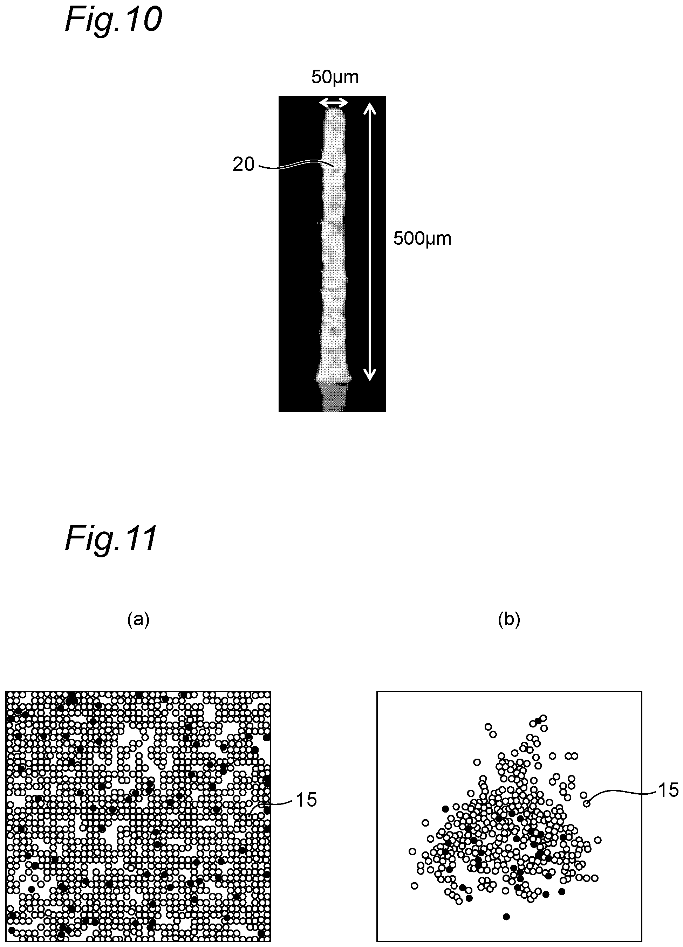

In application experiment 6, by using an application needle 9 (see (c) of Fig. 3) having a tip 9a with a stepped protrusion 9b and a tip diameter of 100 µm, the sodium alginate-containing cell dispersion was consecutively applied 1600 times on a slide glass. At that time, the needle tip was shifted upward by 0.5 µm every cell application operation to produce a cell assembly 20. This resulted in production of the cell assembly 20 with a diameter of 50 µm and a height of 500 µm. Fig. 10 is an image picture showing a cell assembly 20 produced in application experiment 6.

As described above, the cell assembly 20 with a desired shape was found to be able to be produced by repeating multiple cycles of cell application operation while the stop position (return position) of the tip 9a of the application needle 9 was shifted upward (e.g., by 0.5 µm), with respect to a certain application target position (certain point) on the substrate 11, per cycle during the application step in the cell application operation.

### (Experimental Example 7)

In application experiment 7, the viability of cells applied in liquid droplet spots formed by application using the micro-applicator 1 was examined. In application experiment 7, normal human dermal fibroblasts (NHDF) were dispersed at a concentration of 8 × 10⁷ cells/mL in PBS solution to prepare an application liquid 10. In addition, in application experiment 7, this application liquid 10 was consecutively applied 40 times on a slide glass by using the application needle 9 with a tip diameter of 330 µm, and the viability of cells 15 after the application was evaluated by viable cell/dead cell (Live/Dead) fluorescent staining (dead cells were stained red). Fig. 11 is pictures showing viable cell/dead cell (Live/Dead) fluorescently stained images in application experiment 7.

In Fig. 11, (a) shows a viable cell/dead cell (Live/Dead) fluorescently stained image of cells 15 in a pre-application application liquid 10 and (b) shows a viable cell/dead cell (Live/Dead) fluorescently stained image of cells 15 in a post-application liquid droplet spot. Note that in the viable cell/dead cell (Live/Dead) fluorescently stained images in application experiment 7, viable cells were stained green and dead cells were stained red. Here, in the pictures showing the viable cell/dead cell (Live/Dead) fluorescently stained images in Fig. 11, viable cells were denoted by ○ and dead cells were denoted by ●.

The results of examining the cell 15 viability in application experiment 7 demonstrated that the pre-application cell viability was 96% and the post-application cell viability was 91% and still high. This result clearly confirmed that during the cell application operation using the micro-applicator 1, almost no damage was given directly to the cells 15. Note that the pre-application cell viability was slightly dropped from 96% to the post-application cell viability of 91 %. Here, this drop was a regular decrease occurring over time and was caused by another factor.

### (Experimental Example 8)

In application experiment 8, the micro-applicator 1 was used to construct a cellular tissue having iPS cell-derived cardiomyocytes (iPS-CM) on a cell disc. Then, pulsation behavior of a myocardial tissue body in the cellular tissue was assessed.

In application experiment 8, iPS-CM was dispersed at a concentration of 4 × 10⁷ cells/mL in 20 mg/mL fibrinogen solution to prepare an application liquid 10 (first application liquid). In application experiment 8, application was consecutively carried out 10 times on a cell disc by using the application needle 9 with a tip diameter of 330 µm, and the cell disc was then immersed in 800 unit/mL (8.3 mg/mL) thrombin solution (second application liquid) to immobilize a tissue by gelation. Thrombin action caused fibrinogen to form fibrin (blood coagulation-related protein), and this gelatination reaction was utilized to immobilize a tissue on a substrate.

After that, the tissue was soaked in culture medium and cultured for 6 days, and pulsation behavior was recorded over time under a phase-contrast microscope. As a result, immediately after the application, myocardial tissues with a diameter of about 300 µm were formed and even after 6 days of culturing, the structure of equally spaced myocardial tissues was able to be observed.

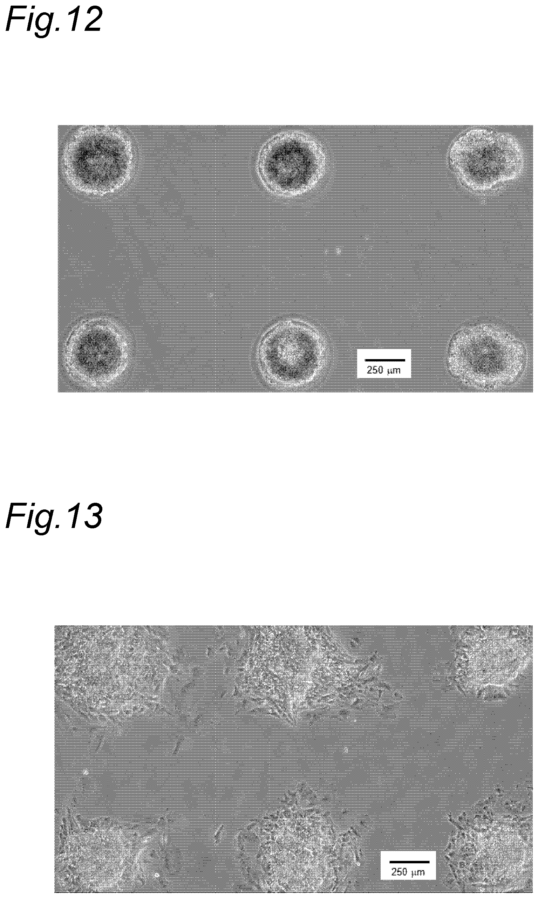

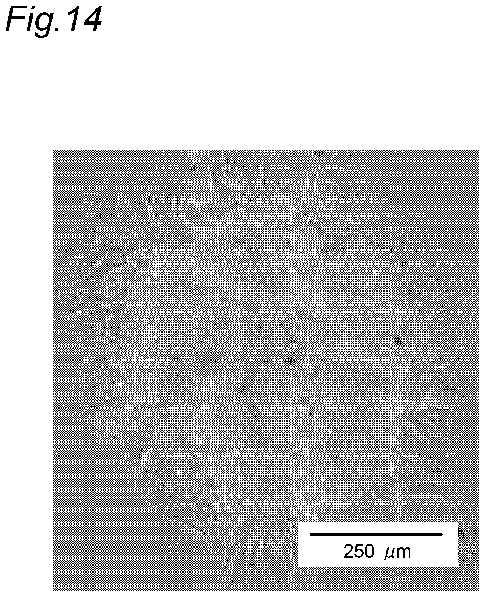

Fig. 12 is a photograph showing states (at culture day 0) of myocardial tissues, produced in application experiment 8, immediately after application. Fig. 13 is a photograph showing states of the myocardial tissues shown in Fig. 12 after 6 days of culturing. Fig. 14 is an enlarged image of one of the myocardial tissues after 5 days of culturing. Figs. 13 and 14 demonstrated that the structure of each myocardial tissue was examined and each cellular tissue was found to be reliably constructed.

In the myocardial tissues produced, cardiomyocytes started pulsation after culture day 2, and at culture day 6, average 82 pulsations per min were recorded in 6 samples. The standard deviation of the 6 samples was 15.

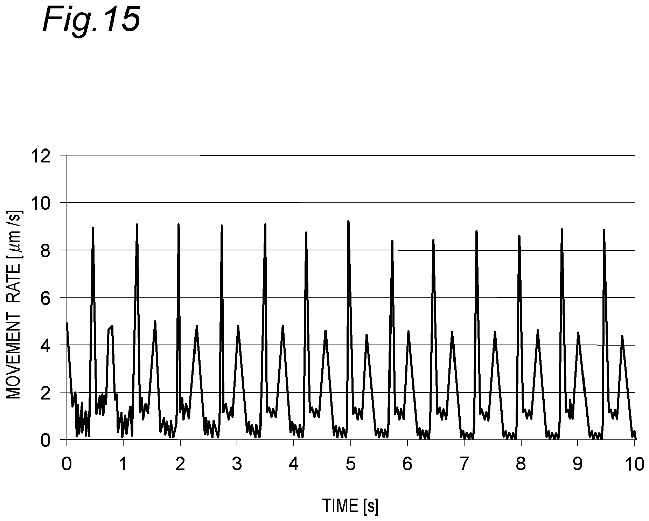

In addition, a pulsation video captured using a high-speed camera was analyzed to calculate a contraction-relaxation rate with a constant cycle. Fig. 15 is a graph showing a contraction-relaxation rate obtained by analyzing a pulsation video of the myocardial tissue (cellular tissue) cultured for 5 days as shown in Fig. 14. In Fig. 15, the ordinate represents a movement rate [µm/s] at which the myocardial tissue contracts and relaxes, and the abscissa represents time [s].

As shown in Fig. 15, the myocardial tissue (cellular tissue) exhibited constant pulsations and the contraction-relaxation rate with a constant cycle was found. At that time, the pulsation rate was 78 times/min, the average contraction rate was 8.7 µm/s, and the average relaxation rate was 4.6 µm/s.

The myocardial tissues obtained in the above application experiment 8 may be produced in respective wells on, for instance, a 96-well plate. This makes it possible to produce a high-throughput cardio-toxicity evaluation kit allowing for automated robotic evaluation under aseptic conditions.

Examples of the cells that can be used in the invention include: but are not particularly limited to, various primary cells such as fibroblasts, vascular endothelial cells, epithelial cells, smooth muscle cells, cardiomyocytes, gastrointestinal cells, neurons, hepatocytes, renal cells, and/or pancreatic cells; differentiated cells derived from stem cells such as ES cells and iPS cells; and various cancer cells. As the cells, it is possible to use: unmodified cells; or cells modified with, for instance, a protein(s), a sugar chain(s), or nucleic acid such as cells coated by a coating procedure with a known coating agent such as fibrinogen, gelatin, collagen, laminin, or elastin.

Note that to give included cells a stable adhesion/proliferation environment, the cell-containing solution may include: an extracellular matrix component(s) such as fibronectin, gelatin, collagen, laminin, elastin, and/or Matrigel; a cell growth factor(s) such as fibroblast growth factor and/or platelet-derived growth factor; or an additional additive agent(s) such as vascular endothelial cells, lymphatic endothelial cells, and/or various stem cells. In addition, for instance, fibrinogen, alginic acid, or a heat-sensitive responsive polymer may be included as a gelatinizer.

As described using the above embodiment 1 and each experimental example, the invention provides a novel production method for producing a cellular tissue. Compared to the case of production using a conventional printer with a nozzle, the invention is configured to use the application needle to apply a solution attached to its tip surface. Thus, clogging of the solution is suppressed and the resolution and the rate of formation of cellular tissue(s) is improved, so that a less sample volume (sample) can be used to definitely produce a highly reliable cellular tissue(s). In addition, compared to the case of using conventional printers, the invention enables a cellular tissue to be produced by applying a highly viscous cell dispersion onto a target. This can suppress evaporation of the cell dispersion after the application, thereby maintaining high cell viability.

Regarding the micro-applicators in the invention, a tiny volume of the application liquid attached to the tip of the needle (application needle) is brought into contact with an application target; and a liquid droplet in an application volume of several pL (picoliter) can be applied with high positional precision, such as positional precision of ±15 µm or less and preferably ±3 µm or less. In addition, it is possible to apply a material with a viscosity of the application liquid of 1 × 10⁵ mPa·s or lower. This allows for application of highly viscous cell dispersion. As such, according to the invention, a highly viscous cell dispersion can be precisely applied at a predetermined position relative to a target (e.g., a substrate). This makes it possible to produce a cellular tissue with a given pattern and three-dimensionally arranged cells. As a result, the produced cellular tissue can be utilized in the fields of regenerative medicine and drug discovery research such as drug efficacy or safety evaluation screening.

### (Embodiment 2)

Hereinafter, embodiment 2, which relates to a method for producing a cellular tissue according to the invention and a cellular tissue prepared by the production method, will be described. Note that, in the description for embodiment 2, elements having substantially the same action, structure, and function as of the above embodiment 1 may have the same reference numerals so as to omit and avoid redundant description.

As described in embodiment 1, the diameter d of the tip 9a of the application needle 9 may be from 50 to 330 µm, and as described in experimental example 2, the droplet spot formed has a droplet spot diameter substantially proportional to the diameter of the application needle 10. As such, the method for producing a cellular tissue of embodiment 1 may be used to form a very small liquid droplet spot. In order to efficiently culture a cellular tissue, cultureware having multiple wells (bottomed wells) is commonly used. In a conventional method for producing a cellular tissue using such cultureware, one cellular tissue per well (bottomed well) was produced and evaluated. However, the micro-applicator 1 in embodiment 1 may be used to form liquid micro-droplet spots. This allows a plurality of cellular tissues to be produced per well (bottomed well).

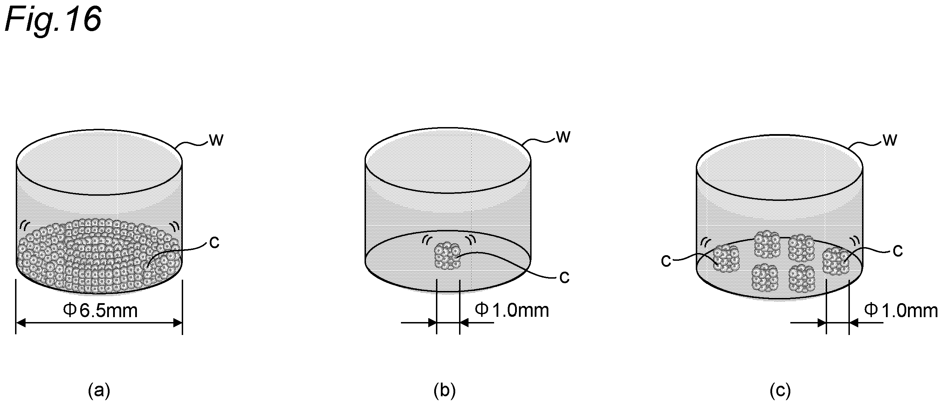

Fig. 16 shows one well w in cultureware having multiple wells (e.g., a 96 well plate), and is diagrams each schematically illustrating a state in which a cellular tissue(s) c is produced on a flat bottom well w with a diameter of 6.5 mm. (a) of Fig. 16 illustrates a state in which a cellular tissue c is produced on the entire one well w, and is a state in which the cellular tissue c is produced by a conventional method for producing a cellular tissue, (b) of Fig. 16 illustrates a state in which one cellular tissue c is produced on one well w using the method for producing a cellular tissue of embodiment 1. (c) of Fig. 16 illustrates a state in which a plurality of (6) cellular tissues c are produced on one well w by using the method for producing a cellular tissue of embodiment 1. Each cellular tissue c produced as illustrated in Fig. 16 is immersed in a culture medium and cultured.

As shown in (c) of Fig. 16, use of the method for producing a cellular tissue of embodiment 1 makes it possible to produce, for instance, 6 cellular tissues c on a flat bottom well w with a diameter of 6.5 mm. The number of cellular tissues that can be produced on one well w varies depending on the size of the well w and the sizes of the cellular tissues.

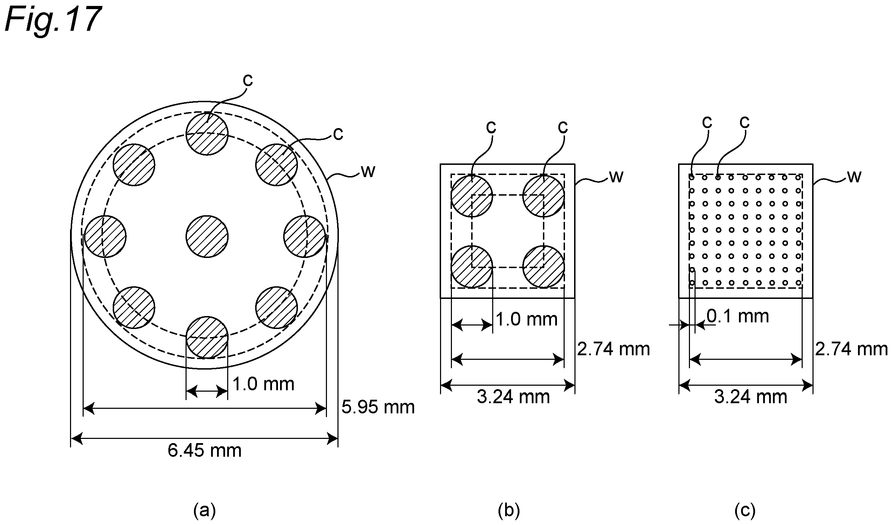

Fig. 17 is plan views each schematically showing an example in which cellular tissues c are produced on a variously shaped well w. In Fig. 17, (a) shows that in a 96 well plate, 8 cellular tissues c can be produced circumferentially on one well w with a diameter of 6.45 mm, and 1 cellular tissue c can be produced in the center thereof. The diameter of the cellular tissue c shown in Fig. 17 (a) is about 1.0 mm. (b) of Fig. 17 illustrates an arrangement example in a case where 4 cellular tissues c with a diameter of about 1.0 mm are produced on a square flat bottom well w of 3.24 mm × 3.24 mm in a 384-well plate. (c) of Fig. 17 illustrates an arrangement example in a case where 81 (9 × 9) cellular tissues c with a diameter of about 0.1 mm are produced on a square flat bottom well w of 3.24 mm × 3.24 mm in a 384-well plate. In the arrangement example of each cellular tissue c shown in Fig. 17, the distance from the inner wall surface of the well w to the cellular tissue c is 250 µm.

As described above, the method for producing a cellular tissue of embodiment 1 may be used to produce a plurality of cellular tissues c inside a small well w. The method for producing a cellular tissue of embodiment 1 makes it possible to produce cellular tissues c at an arrangement density of 771.4 cells/cm².

As described above, a plurality of cellular tissues c may be produced on one small well w to evaluate multiple samples in one well w. In addition, a plurality of samples can be imaged by one imaging during analysis of an image or a video. This can shorten the imaging time and can thus shorten analysis time. For instance, in the case of using a 96-well w as cultureware, only one sample can be produced in one well w by a conventional method for producing a cellular tissue. However, the method for producing a cellular tissue of embodiment 1 makes it possible to produce 9 cellular tissue samples c with a diameter of about 1.0 mm on one well w. Thus, the method for producing a cellular tissue of embodiment 1 makes is possible to produce 864 (96 × 9) cellular tissue samples c by using 96-well w cultureware. In addition, for instance, 20 sec of imaging may be required for video analysis of one well w. This case needs 180 sec for video imaging of 9 samples because in a conventional method for producing a cellular tissue, one well w has one sample. By contrast, in the method for producing a cellular tissue of embodiment 1, 9 samples are produced inside one well w, so that the video imaging is greatly shortened to 20 sec.

### (Embodiment 3)

Hereinafter, embodiment 3, which relates to a method for producing a cellular tissue according to the invention and a cellular tissue prepared by the production method, will be described with reference to the accompanying drawings. Note that, in the description for embodiment 3, elements having substantially the same action, structure, and function as of the above embodiment 1 or 2 may have the same reference numerals so as to omit and avoid redundant description.

In the method for producing a cellular tissue according to embodiment 3 of the invention, the micro-applicator 1, described in detail in the above embodiment 1, is used and configured such that a cell-containing application liquid (first application liquid) attached to the tip 9a of the application needle 9 can be applied. In experimental example 8 in the above embodiment 1, the micro-applicator 1 was used to apply the application liquid 10 (first application liquid) in which iPS cell-derived cardiomyocytes (iPS-CM) had been dispersed in a fibrinogen solution, and the liquid was then immersed in a thrombin solution (second application liquid) to immobilize a tissue by gelation.

As described above, after the iPS cell-derived cardiomyocytes were dispersed in the fibrinogen solution and applied by contact application using the application needle 9, the iPS cell-derived cardiomyocytes were immersed in the thrombin solution to immobilize the tissue by gelation. In this production method, when fibrinogen is used as a gelatinizer at the time of applying cardiomyocytes, cardiomyocytes and fibrinogen are dispersed in a first application liquid as a first liquid and applied, and thrombin as a gelling initiator is added to a second application liquid as a second liquid so as to cover the first application liquid. In this way, the application operations are carried out.

That is, the method includes:
a first application step of applying a first application liquid containing cells (e.g., cardiomyocytes) and a gelatinizer (e.g., fibrinogen) to an application target; and
a second application step of applying and layering a second application liquid containing a gelling initiator (e.g., thrombin) onto the first application liquid applied in the first application step to start a gelation reaction. In this way, the method for producing a cellular tissue is implemented. Gelation starts at an interface between the first application liquid and the second application liquid. Note that this production method is called "two-component cellular tissue production method of embodiment 1". The following two-component cellular tissue production method in embodiment 3 is called "two-component cellular tissue production method of embodiment 3".

In the two-component cellular tissue production method of embodiment 1, a phenomenon was observed in which unintended gelation occurred in the solution containing the cells (cardiomyocytes) and the gelatinizer (fibrinogen) inside the first application liquid before the application step was executed. Thus, the cardiomyocytes gelled in the application liquid container remained in the application liquid container, and all the application liquids inside the application liquid container could not be applied in some cases.

The method for producing a cellular tissue of embodiment 3 can prevent the occurrence of gelation inside the application liquid container before the application step is executed. Also in the method for producing a cellular tissue of embodiment 3, a two-component gelling solution is used as application liquids at the time of cellular tissue formation (hereinafter, referred to as "two-component cellular tissue production method of embodiment 3").

In the two-component cellular tissue production method of embodiment 1, the cell-containing first solution contains a gelatinizer, and the second application solution contains a gelling initiator. By contrast, in the two-component method for producing a cellular tissue of embodiment 3, the cell-containing first solution contains a gelling initiator, and the second application solution contains a gelatinizer. Other than this point, both share and are configured by the concepts and elements.

Embodiment 3 relates to a method for producing a cellular tissue including:
a first application step of applying a first application liquid containing a cell(s) and a gelling initiator to an application target; and
a second application step of applying and layering a second application liquid containing a gelatinizer onto the first application liquid applied in the first application step to start a gelation reaction.

In addition, embodiment 3 relates to a cellular tissue production set including a first container containing a first application liquid containing a cell(s) and a gelling initiator and a second container containing a second application liquid containing a gelatinizer.

First, the method for producing a cellular tissue according to embodiment 3 of the invention will be described, including
a first application step of applying a first application liquid containing a cell(s) and a gelling initiator to an application target; and
a second application step of applying and layering a second application liquid containing a gelatinizer onto the first application liquid applied in the first application step to start a gelation reaction.

### First Application Step

The cells to which the method of the invention is applicable are not particularly limited, and are cells such as iPS cell-derived cardiomyocytes, normal human cardiac fibroblasts, normal human fibroblasts, human vascular endothelial cells, or HePG2 cells. Two or more of them may be mixed and used. Among them, the invention is preferably applied to iPS cell-derived cardiomyocytes, normal human fibroblasts, or HepG2 cells, and particularly iPS cell-derived cardiomyocytes. In the case of using iPS cell-derived cardiomyocytes, they are brought closer to the morphology of the heart of a living body, and the iPS cell-derived cardiomyocytes are matured by factors secreted from normal human cardiac fibroblasts. Because of this, the normal cardiac fibroblasts are usually mixed therewith and used. Examples of the cell(s) that can be used include an unmodified cell(s) or a cell(s) modified with, for instance, a protein(s), a sugar chain(s), or nucleic acid, such as a cell(s) coated by a coating procedure with a known coating agent such as fibronectin, gelatin, collagen, laminin, elastin, or Matrigel.

As the gelling initiator contained in the first application liquid, thrombin, calcium chloride, or an alcohol compound (e.g., ethyl alcohol, glycerin) may be used. These gelling initiators are usually selectively used depending on the type of gelatinizer contained in the second application liquid. The selective use is known to those skilled in the art, and they may be used in such known combination. Examples of the combination of a gelling initiator and a gelatinizer used (gelling initiator: gelatinizer) include a combination of thrombin : fibrinogen (gelling initiator: gelatinizer), calcium chloride : sodium alginate (gelling initiator: gelatinizer), calcium chloride : carrageenan (gelling initiator: gelatinizer), or an alcohol compound : tamarind seed gum (gelling initiator: gelatinizer).

When iPS cell-derived cardiomyocytes are used as the cells, a combination of thrombin and fibrinogen (gelling initiator: gelatinizer) is preferably used.

In the first application liquid, the cell(s) and the gelling initiator are contained in an aqueous solution.

Examples of the aqueous solution used include water or various kinds of physiological saline such as phosphate buffered saline or Tris buffered saline. In addition, examples of a culture medium used as a cell culture liquid include Dulbecco's Modified Eagle Medium. These aqueous solutions are usually used selectively depending on the type of cells. The selective use is known to those skilled in the art, and they may be used in such known combination. For example, in the case of iPS cell-derived cardiomyocytes, it is preferable to use phosphate buffered saline (PBS), Dulbecco's Modified Eagle Medium, or a special medium from each company, and particularly phosphate buffered saline.

The amount of cells contained in the aqueous liquid container is not particularly limited, and the cells may be included at a concentration of from about 1 x 10⁵ cells/mL to 1 x 10⁹ cells/mL, preferably from about 1 x 10⁶ cells/mL to 1 x 10⁸ cells/mL, and more preferably from about 1 x 10⁷ cells/mL to 1 x 10⁸ cells/mL. If the amount is too large, it is difficult to adjust the solution, and if the amount is too small, no cells are included depending on the application amount.

The content of the gelling initiator is not particularly limited, and usually, the content may be set, if appropriate, in view of the rate of gelation. If the amount is too small, the rate of gelation is slow, and the shape retainability in the first application liquid, which is the first liquid, is lowered. For example, when thrombin is used, the thrombin may be included at a concentration of from about 1 unit/mL to 1000 unit/mL, preferably from 10 unit/mL to 1000 unit/mL, and more preferably from 100 unit/mL to 800 unit/mL.

In order to give cells a stable adhesion/proliferation environment, the first application liquid optionally contains: an extracellular matrix component(s) such as fibronectin, gelatin, collagen, laminin, elastin, and/or Matrigel; a cell growth factor(s) such as fibroblast growth factor and/or platelet-derived growth factor; or an additive agent(s) such as vascular endothelial cells, lymphatic endothelial cells, and/or various stem cells.

A thickener may be further added to the first application liquid as an additive agent. As the thickener, it is possible to use a thickening polysaccharide such as sodium hyaluronate or sodium alginate.

The thickener is included in order to impart shape retainability to the first application liquid, and in particular, the thickening polysaccharide is effective in creating a three-dimensional cellular tissue having high shape retainability.

When the thickener is included, the amount thereof may be set, if appropriate, in consideration of, for instance, the shape retainability in the first application liquid, the possible viscosity range of the applicator used, cost, and handling performance. For example, in the application process using the micro-applicator 1 in embodiment 1, a thickener may be included so as to have a viscosity of from 1 mPa·s to 1 × 10⁵ mPa·s, preferably from 3 × 10³ mPa·s to 1 × 10⁵ mPa·s, and more preferably from 1 × 10⁴ mPa·s to 5 × 10⁴ mPa·s. If the viscosity is too low, the shape retainability in the first application liquid is deteriorated. Note that as the viscosity of the first application liquid, a value measured at 25°C by a rotational viscosity method is used.

Extracellular matrix is included to promote the growth of cells and promote adhesion between cells or between cells and a substrate. When the extracellular matrix is included, the amount thereof is not particularly limited. The type and concentration may be designated according to a tissue to be produced.

The first application step in the method for producing a cellular tissue of embodiment 3 includes applying a first application liquid to an application target. The application target used is not particularly limited, and cultureware usually used for growth of cells is used. Examples of the "identical cultureware (well)" used in the invention include a 6- to 384-well plate, a dish, a petri dish, or a MicroWell plate (Registered trademark, Thermo Fisher Scientific Inc.). The "cultureware" used herein is a recessed well(s) having a flat or curved bottom surface and having a side surface. In addition, the "cultureware" may have a hemispherical surface formed by a curved surface where a bottom surface and a side surface are continuous. Note that the "multi-well cultureware" is integrated cultureware having a plurality of recessed wells (recessed parts: wells).

The application process in the method for producing a cellular tissue of embodiment 3 is not particularly limited to the micro-applicator 1. Examples of the process that can be used include a known bioprinting technique such as an inkjet technique, a dispenser technique, or a laser assisted technique.

When the viscosity of the solution is high, the application needle procedure using the micro-applicator 1 described in embodiment 1 may be suitably used. For a process of applying a solution or a liquid by using the application needle procedure, the micro-applicator 1 described in embodiment 1 may be used. In addition, it is possible to use, for instance, the applicator disclosed and described in Patent Document 10 (JP 4802027 B) or Patent Document 11 (WO 2019/088224). The entire contents disclosed and described in the specifications and drawings are cited herein as part of the disclosure herein.

For instance, the application needle procedure using the micro-applicator 1 described in embodiment 1 can be implemented by using an application liquid with a broad range of viscosity for micro-application. During application operation, a single application needle is protruded from a through-hole formed at the bottom of an application liquid container that retains the application liquid. The application needle procedure is implemented for application such that the application liquid attached to the tip is brought into contact with an application target.

In the process of applying the first application liquid, the micro-applicator 1 described in embodiment 1 is used. In the application process using the micro-applicator 1, as described above, the tip 9a of the application needle 9, to which an extremely small amount of application liquid is attached, may be brought into contact with a target (e.g., a substrate) to apply a droplet having an application amount of several pL (picoliter) to several 100 µL (microliter) with high arrangement accuracy, for example, arrangement accuracy of ±15 µm or less and preferably ±3 µm or less. In addition, it is possible to apply a material with a viscosity of the first application liquid of 1 × 10⁵ mPa·s or lower. This allows for application of highly viscous cell dispersion. As such, the application process using the micro-applicator 1 makes it possible to precisely apply the high-viscosity cell dispersion to a predetermined position on an application target (e.g., a substrate). In particular, an application liquid having a viscosity of about 3 × 10³ mPa·s or higher may be used to apply the application liquid three-dimensionally, specifically, like a three-dimensional dome. Also, it is possible to retain the three-dimensional shape until the next second application step. As used herein, this property is referred to as "shape retainability".

The application amount of the first application liquid may be set, if appropriate, in view of the application process conditions depending on the desired amount. In the application process using the micro-applicator 1, the amount may be adjusted by, for instance, the diameter of the tip 9a of the application needle 9 or the number of times of application.

More specifically, in the application process using an application needle, a micro-applicator used is provided with an application unit including:
an application liquid container having an application liquid reservoir that stores a predetermined amount of a first application liquid; and
an application needle capable of penetrating the application liquid reservoir in which the first application liquid is stored. The application process executed includes:
   a waiting step of dipping a tip of an application needle into a first application liquid charged in an application liquid reservoir;
   a descending step of causing the tip of the application needle to penetrate through the application liquid reservoir and causing the tip of the application needle having the first application liquid attached to descend;
   an application step of bringing the tip of the application needle having the first application liquid attached into contact with an application target and applying the first application liquid onto the application target to form a liquid droplet spot; and
   a holding step of lifting the tip of the application needle and holding the tip of the application needle in the application liquid reservoir.

Even in the case of using a bioprinting technique other than the application needle procedure, the application may be performed by adjusting the application conditions, if appropriate, so as to obtain an applied material like in the application needle procedure set under appropriate application conditions.

### Second Application Step

In the second application liquid used in the second application step, the gelatinizer is included in an aqueous solution.

As the gelatinizer included in the second application liquid, for instance, fibrinogen or sodium alginate may be used. These gelatinizers are usually used selectively depending on the type of gelling initiator included in the first application liquid. The selective use and combinations have been described in the above first application liquid section.

As the gelatinizer included in the second application liquid, it is possible to use a gelatinizer that cures without any gelling initiator. When such a gelatinizer is used, the first application liquid does not necessarily contain a gelling initiator. Examples of such a gelatinizer include: collagen, gelatin, agar (agarose); or a photocrosslinkable gelatin hydrogelatinizer such as gelatin-methacrylamide or gelatin-acrylamide. For instance, collagen is gelatinized by heating, and gelatin is gelatinized by cooling. When the second application liquid containing such a gelatinizer is used, the second application liquid is applied and is then heated in the case of containing collagen. In the case of containing gelatin, a cooling operation should be performed to make a gelation reaction. Gelatin-methacrylamide is subject to gelation when irradiated with ultraviolet light (at a wavelength of 365 µm). Gelatin-acrylamide is subject to gelation when irradiated with ultraviolet light (at a wavelength of 365 µm). In the case of using the second application liquid containing such a photocrosslinkable gelatin hydrogelatinizer, it is necessary to apply the second application liquid and then make a gelation reaction by irradiation with light (e.g., UV light, visible light).

As the aqueous solution contained in the second application liquid, it is possible to use substantially the same aqueous solution as used for the above-mentioned first application liquid. The aqueous solution used may be the same aqueous solution used for the first application liquid or may be a different kind of aqueous solution.

The amount of the gelatinizer included in the aqueous solution is not particularly limited, and the content thereof may be set, if appropriate, in consideration of, for instance, the rate of gelation, the concentration of the gelatinizer, or the hardness of the gel. If the amount is too small, the rate of gelation is slow. For instance, in the case of using fibrinogen, the concentration thereof may be set to a concentration of from 0.1 mg/mL to 100 mg/mL, preferably from 1 mg/mL to 80 mg/mL, and more preferably from 1 mg/mL to 30 mg/mL.

The second application liquid optionally contains a component(s) (further additive(s)) such as a thickener. The thickener is included from the viewpoint of shape retention in the applied first application liquid, and, for instance, sodium hyaluronate or sodium alginate may be used. In the case of including the thickener, the amount thereof may be set, if appropriate, in consideration of, for instance, the shape retainability in the applied first application liquid, the possible viscosity range of the micro-applicator 1 used, cost, or handling performance. For instance, in the application process using the application needle 9, the second application liquid may contain a thickener so as to have a viscosity of from 5 × 10² mPa·s to 1 × 10⁵ mPa·s, preferably from 5 × 10² mPa·s to 1 × 10⁴ mPa·s, and more preferably from 5 × 10² mPa·s to 5 × 10⁴ mPa·s. If the amount is small and the viscosity is too low, the shape retainability in the applied first application liquid cannot be maintained. Note that as the viscosity of the second application liquid, a value measured at 25°C by a rotational viscosity method is used.

Regarding the second application liquid, the second application liquid is applied and layered onto the first application liquid applied in the first application step. The term "layering" means that application is performed so as to cover the first solution applied on an application target (e.g., a substrate) in the first application step, and includes meaning that a surface other than a surface in contact with the first solution on the application target is also covered.

As described above, when the second application liquid is applied and layered onto the first application liquid, an interface between the applied first solution and the second solution is subject to gelation. At this time, the inside of the first application liquid present under the gelled interface is not subject to gelation, and the solution state (viscosity) of the first solution is maintained. Thus, the cells inside the first application liquid are settled and precipitated by gravity and aggregated, deposited, and layered in a lower portion of the first application liquid.

The time interval from the end of the first application step to the start of the second application step is desirably as short as possible. If the time interval is too long, there arises a problem that the first application liquid is dried and the contained cells die.

The application amount of the second application liquid is set, if appropriate, in consideration of, for instance, the application amount of the first application liquid and the rate of gelation such that the second application liquid is applied and layered onto the first application liquid.

The process of applying the second application liquid may be an application process using the micro-applicator 1 in embodiment 1. Examples of the process that can be used include, but are not particularly limited to, manual application such as liquid droplet application by, for instance, a syringe or a dropper or a bioprinting technique such as an inkjet process or a dispenser process.

After the second application liquid is applied, only a contact surface (interface) between the first application liquid and the second application liquid is subject to gelation. A curing initiator is present, as a liquid, inside the first application liquid existing under the gelled interface. The solution state (viscosity) of the first application liquid is maintained. The cells are settled and precipitated over time, and aggregated, deposited, and layered in a lower portion of the first application liquid. This procedure can be used to produce a three-dimensional cellular tissue having high density and high shape retainability without any complicated production step.

The contact surface between the first application liquid and the second application liquid, which surface is the outer peripheral portion of the cellular tissue immediately after application, is subject to gelation. Then, the inside of the first application liquid is kept in a liquid state. This can also prevent the cellular tissue from being dried.

The cells are cultured by immersing, in a culture medium, the application liquids applied in the first application step and the second application step. At this time, the application target (substrate) may also be immersed together in the culture medium. As the culture medium, a commonly used culture medium may be used in consideration of cells. For instance, in the case of including iPS cell-derived cardiomyocytes as the cells, Dulbecco's Modified Eagle Medium or a commercially available special culture medium from each company is used.

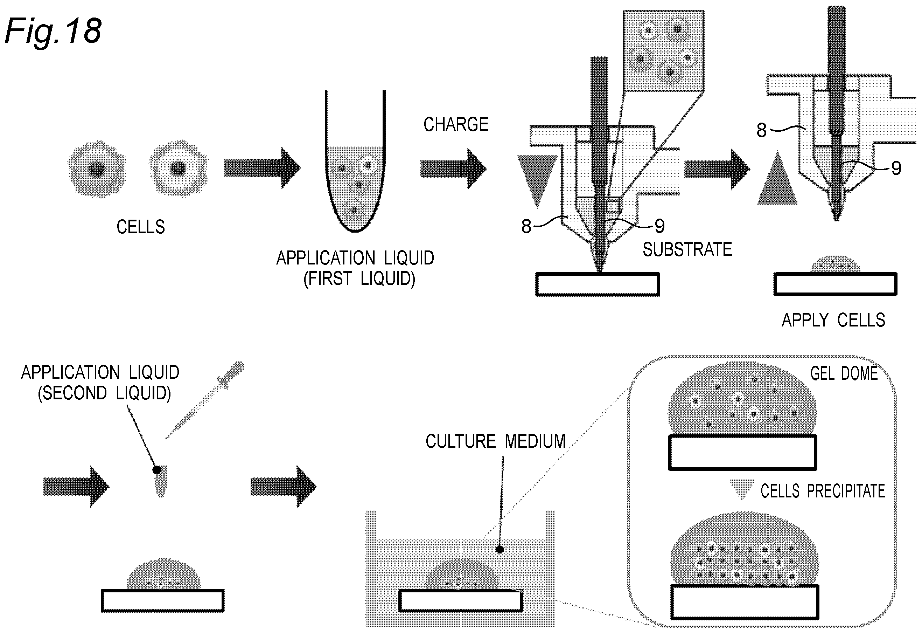

Fig. 18 is a diagram schematically illustrating the outline of the cellular tissue production method performed in embodiment 3, and is a schematic diagram showing how a cellular tissue is three-dimensionally assembled after the application step.

A first application liquid containing cells and a gelling initiator in suspension is prepared, and the first application liquid is charged in an application liquid container. The first application liquid (first liquid) is applied to an application target by using the application needle 9. In the first application step of applying the first application liquid, an application operation is repeated a desired number of times. Next, a second application liquid (second liquid) containing a gelatinizer is applied so as to cover the first application liquid (first liquid) applied in the first application step. A culture medium is added after a contact surface between the first application liquid and the second application liquid and the outer peripheral region thereof are subject to gelation. The inside of the first liquid, namely the first application liquid is kept in a liquid state while the fluidity is maintained to give a dome structure. Thus, the cells are settled and precipitated over time, and aggregated, deposited, and layered in a lower portion of the first application liquid. This results in production of a high-density three-dimensional cellular tissue.

As used herein, the term "high density" means a state in which cells are assembled without, for instance, any fibrin gel between cells, and means a case of having a density of from about 1 × 10⁸ cells/mL to 1 × 10⁹ cells/mL when numerically expressed as a cell density.

A second aspect of embodiment 3 according to the invention relates to a cellular tissue production set including at least a first container containing a first application liquid containing a cell(s) and a gelling initiator and a second container containing a second application liquid containing a gelatinizer.

This cellular tissue production set is suitable for use in the method for producing a cellular tissue of embodiment 3. The first application liquid and the second application liquid defined in the cellular tissue production set have the same meaning as the first application liquid and the second application liquid described in the method for producing a cellular tissue of embodiment 3.

According to the method for producing a cellular tissue of embodiment 3, for instance, gelation does not occur inside the application liquid container before the application step is executed in the application step using the micro-applicator 1. In addition, according to the method for producing a cellular tissue of embodiment 3, it is possible to produce a cellular tissue having high density and high shape retainability while the cell viability is maintained.

Hereinafter, the method for producing a cellular tissue of embodiment 3 will be described using specific examples. However, the invention should not be construed such that the invention is limited to those examples. It should be understood that the invention includes specific embodiments and any technical ideas that can be conceived by those skilled in the art who read the concept of the invention and can be considered to be put into practice.

### [Examples]

### Example 1

### (1) Application Process

### (1-1) Operation of Applying First Application Liquid (First Liquid)

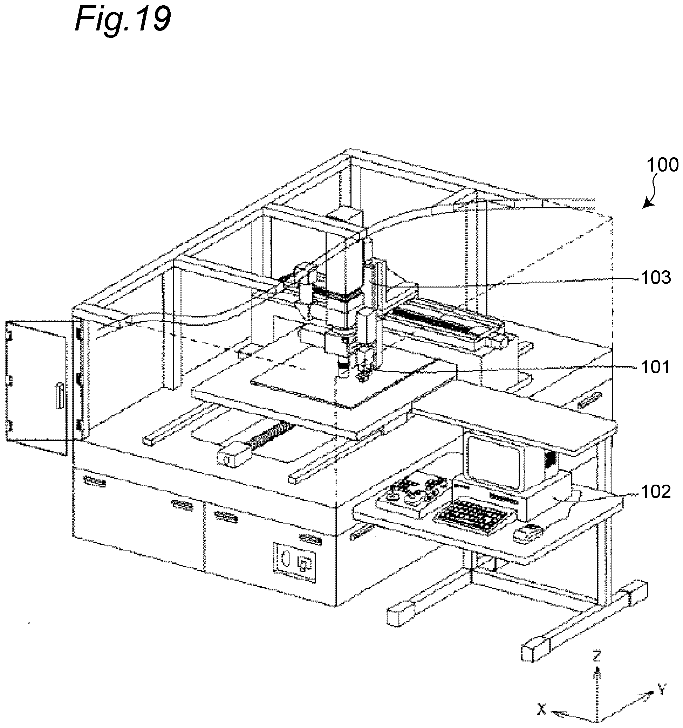

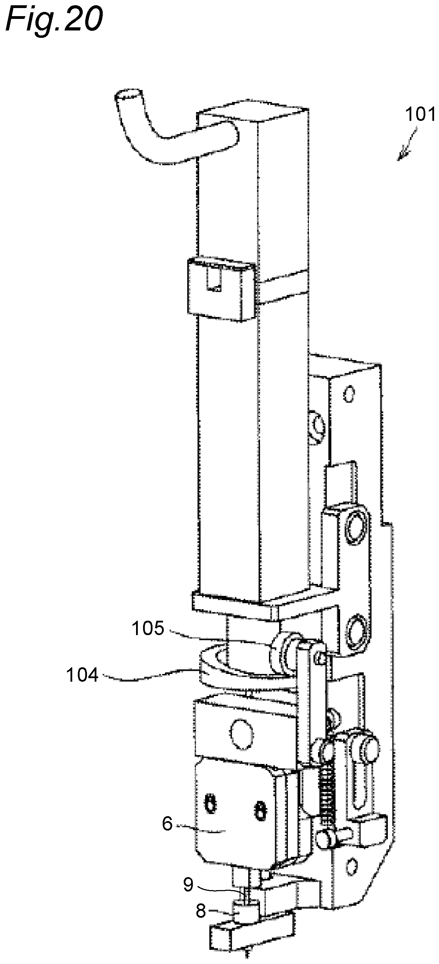

Fig. 19 shows a micro-applicator 100 for a first application liquid used in example 1. Fig. 19 is a perspective view illustrating the micro-applicator 100. The micro-applicator 100 used in example 1 is configured to execute substantially the same application process as for the micro-applicator 1 described in embodiment 1, and is configured to subject the first application liquid attached to the tip 9a of the application needle 9 to contact application on an application target. Fig. 20 is a perspective view illustrating an applicator main body 101 of the micro-applicator 100 used in example 1.

In the applicator main body 101 supported by an XYZ stage 105, the movable part 105 reciprocates in the Z direction by moving a cam 104. Thus, the application needle 9 supported so as to be able to penetrate through the application liquid container 8 reciprocates likewise in the Z direction. This reciprocating operation allows the first application liquid attached to the tip 9a of the application needle 9 is brought into contact with and applied onto the application target.

The display/control unit 103 includes a monitor, a control computer, and an operation panel. The application speed command value is input from the operation panel and stored in a storage device of the control computer. During the application operation, the application speed command value is read from the storage device and sent to a control program of the applicator main body 101. The control program determines a rotational speed of a motor, which is a driving mechanism in the applicator main body 101, based on the application speed command value, and executes the application operation by reciprocating the application needle 9 at a predetermined speed. In the case where the control computer communicates with a host control system (not shown), the application speed command value may be received from the host control system. In addition, a parameter corresponding to the type of the first application liquid may be stored in a memory, and the application speed command value may be calculated according to the designated type of the first application liquid and the application amount or application size.

As described for the micro-applicator 1 in embodiment 1, the tip 9a of the application needle 9 protrudes from a lower hole 14b provided at the bottom of the application liquid container 8 to perform the application operation (see Fig. 4). When the tip 9a of the application needle 9 protrudes from the lower hole 14b of the application liquid container 8, the tip 9a of the application needle 9 has the first application liquid attached and protrudes from the application liquid container 8. At this time, the first application liquid is pulled upward by surface tension, and substantially a constant amount of the first application liquid remains at the tip 9a of the application needle 9. The first application liquid left at the tip 9a of the application needle 9 may be transferred to the application target, so that a reproducible application operation can be realized.

### (1-2) Operation of Applying Second Solution

The operation of applying the second solution was performed by manual dropping using a micropipette.

### (2) Application Conditions: Cells

| | |
|---|---|
| Application needle diameter: | ϕ1000 µm |
| Application needle shape: | Straight |
| Number of times of application: | 10 times |
| Application amount of first application liquid: | Several 100 nL/10 times |
| Application amount of second application liquid: | 15 µL per application |
| Waiting time in the application liquid container: | 1020 ms |
| Waiting time after application needle descent: | 0 ms |
| Lifting time: | 5 µm per application |

Time required until addition of second application liquid: Within 5 s

Time required until a culture medium was added: Culture medium was added manually by using a micropipette after all four pieces had been produced.

### (3) Application Liquid

### (3-1) First Application Liquid (First Liquid)

| | |
|---|---|
| iPS cell-derived cardiomyocytes: | Human cardiac fibroblasts = 3 : 1 |
| Cell count: | 8 x 10⁷ cells/mL |
| Sodium hyaluronate: | 13 mg/mL |
| Thrombin: | 800 unit/mL |
| PBS | |
| Viscosity: | 1.5 × 10⁴ mPa·s) |

### 3-2) Second Application Liquid (Second Liquid)

| | |
|---|---|
| Amount added: | 15 µL |
| Sodium hyaluronate: | 0.5 mg/mL |
| Fibrinogen: | 10 mg/mL |
| PBS | |
| Viscosity: | 1 × 10³ mPa·s |

### (4) Results and Discussion

### (4-1) Production Method

The first application liquid was repeatedly applied multiple times under the above application conditions, and it was found that cardiomyocytes and thrombin as a gelling initiator in the application liquid, which was the first liquid, of the first application liquid container were completely applicable. After the end of the application, the inside of the application liquid container was visually inspected, and no gel of the first application liquid was found to be generated. The second application liquid, which was a second liquid containing fibrinogen as a gelatinizer, is added so as to cover the first application liquid as the first liquid.

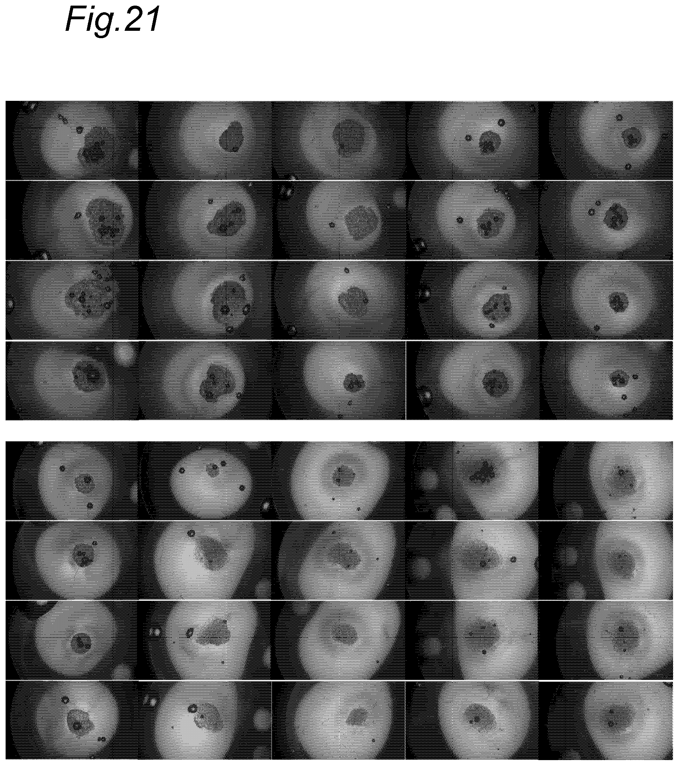

Fig. 21 (magnification: 2) shows a photograph of the application state as a result of application to 40 wells in a 96-well plate under the above conditions, when viewed from the above of the well plate. As shown in Fig. 21, there was almost no variation in the number of cells even when the application was repeated multiple times.

### (4-2) Observation of Tissue

### (4-2-1) Density

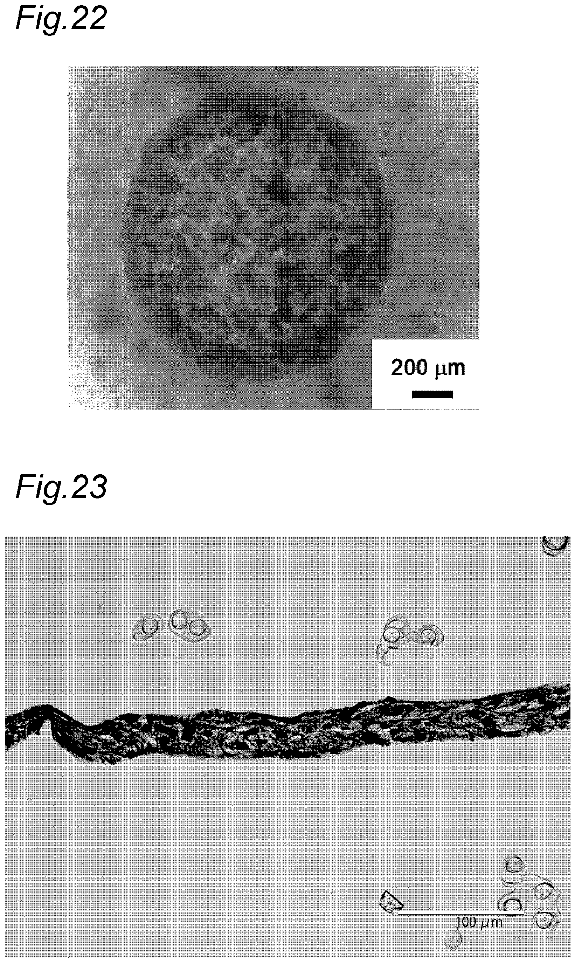

The cellular tissue obtained 1 h after application was microscopically observed using a phase contrast microscope. The microscopy was conducted by observing the application liquid (a state described as a gel dome drawn in the lower right of Fig. 18) from above. Fig. 22 shows the photograph.

As can be seen from Fig. 22, there was almost no intercellular matrix between the cells, and a high-density three-dimensional cellular tissue was found to be produced. The density was 3.5 × 10⁸ cells/mL.

Note that the culture was carried out in an environment at a temperature of 37°C and 5% CO₂ for 7 days.

### (4-2-2) Thickness

The thickness of a cellular tissue obtained after culturing was measured by hematoxylin/eosin (HE) staining of cell nuclei and the cytoplasm. The measured results of hematoxylin/eosin (HE) staining are shown in Fig. 23. Fig. 23 demonstrated that the thickness of this tissue was about 50 µm.

### (4-2-3) Cellular Tissue

Cell nuclei, actin, and troponin were stained.

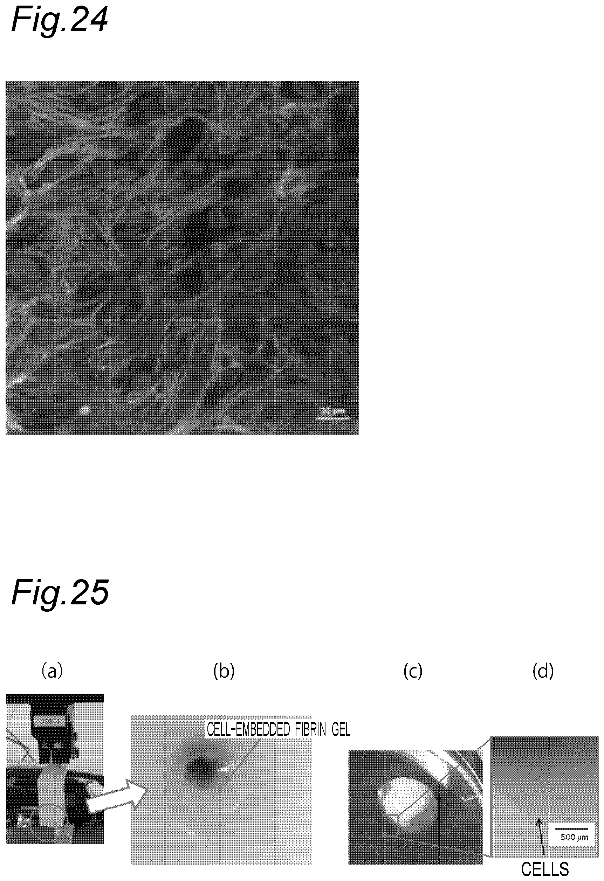

The results of the fluorescent staining are shown in Fig. 24.

This photograph is originally a color photograph, and the cell nuclei were stained blue, the actin was stained red, and the cardiac troponin T was stained green. Fig. 24 demonstrated expression of the striated structure of cardiac troponin T characteristic of cardiomyocytes. The beating rate was from 50 to 60 beats/min, which was close to that of the adult heart.

### (4-3) Discussion

This application process included: applying, multiple times, the first application liquid in which cardiomyocytes and a gelling initiator thrombin had been dispersed as the first liquid; and applying the second application liquid containing a gelatinizer fibrinogen as the second liquid so as to cover the first liquid, namely the first application liquid. This application process made it possible to easily produce a three-dimensional cellular tissue with high density and high shape retainability while the cell viability is maintained without any complicated production step.

### Comparative Example 1

(1) The same micro-applicator 100 used in example 1 was used to apply an application liquid under the following application conditions.

In the comparative example, a gelatinizer (fibrinogen) was included in a first solution and a gelling initiator (thrombin) is included in a second solution. This point significantly differed from the point of example 1.

### (2) Application Conditions

| | |
|---|---|
| Application needle diameter: | ϕ1000 µm |
| Application needle shape: | Straight |
| Number of times of application: | 10 times |
| Application amount of first application liquid: | Several nL/10 times |
| Application amount of second application liquid: | 15 µL per application |
| Waiting time in the application liquid container: | 1020 ms |
| Waiting time after application needle descent: | 0 ms |
| Lifting time: | 5 µm per application |

Time required until addition of second application liquid: Within 5 s

Time required until a culture medium was added: Culture medium was added manually by using a micropipette after all four pieces had been produced.

### (3) Application Liquid

### (3-1) First Application Liquid (First Liquid)

| | |
|---|---|
| iPS cell-derived cardiomyocytes: | Human cardiac fibroblasts = 3 : 1 |
| Cell count: | 8 x 10⁷ cells/mL |
| Sodium hyaluronate: | 13 mg/mL |
| Fibrinogen: | 10 mg/mL |
| PBS | |
| Viscosity: | 1.5 × 10⁴ mPa·s) |

### (3-2) Second Application Liquid (Second Liquid)

| | |
|---|---|
| Amount added: | 15 µL |
| Sodium hyaluronate: | 0.5 mg/mL |
| Thrombin: | 800 unit/mL |
| PBS | |
| Viscosity: | 1 × 10³ mPa·s |

### (4) Results and Discussion

### (4-1) Observation of Tissue

### (4-1-1) Density

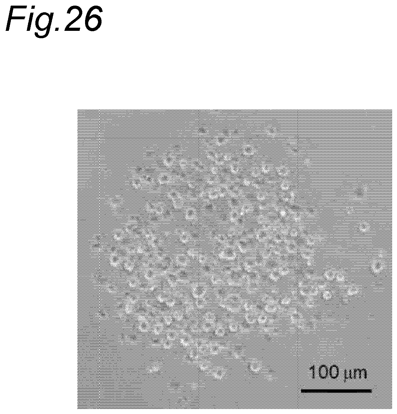

The cellular tissue obtained immediately after application was microscopically observed using a phase contrast microscope. The photograph is shown in Fig. 26.

In Fig. 26, it can be seen that an intercellular matrix exists between substantially spherical cells. It can be found that in a state before the cells immediately after application precipitate, the cell density is lower in Fig. 26 than in Fig. 22. This may be because the cells are immobilized in a sparse state due to the gelatinizer mixed in the first liquid, and as a result of which precipitation does not occur.

### (4-2) Production Method

When cardiomyocytes and fibrinogen were dispersed in the first application liquid as the first liquid, a gel-like substance, in which cardiomyocytes were embedded, in the application liquid container was identified after 15 min had passed (Fig. 25).

(a) of Fig. 25 shows a specific application needle 9 and a specific application liquid container 8 supported so as to penetrate the application needle therethrough, and (b) of Fig. 25 shows a cell-embedded fibril gel formed around a lower hole 14b of the application liquid container 8. (c) of Fig. 25 shows the cell-embedded fibril gel taken out from the application liquid container 8. (d) of Fig. 25 is an enlarged view of (c) of Fig. 25.

In the production method of this comparative example, cells remained in the application liquid container, and the number of cells in the first application liquid varied.

### (4-3) Discussion

This application process included: applying the first application liquid in which cardiomyocytes and fibrinogen had been dispersed as the first liquid; and applying a gelling initiator, i.e., the second application liquid containing thrombin as the second liquid so as to cover the first application liquid. In this comparative example, since fibrinogen was subject to gelation in the form of embedding cardiomyocytes, the cardiomyocytes were sparsely dispersed in the gel. This caused the problem that made it difficult to easily produce a three-dimensional cellular tissue with high density and high shape retainability while the cell viability is maintained.

Further, the first application liquid in which cardiomyocytes and fibrinogen had been dispersed as the first liquid was repeatedly applied. Alternatively, the first application liquid was retained in the application liquid container for a long period of time. In these cases, gelation of the first application liquid occurred in the application liquid container. This has caused the problem that a cardiomyocyte mass gelled inside the application liquid container remains in the application liquid container and cannot be applied in some cases.

From the above disclosure, for example, the following is provided as more specific embodiments in the first aspect of embodiment 3 according to the invention.
(1) A method for producing a cellular tissue, including: a first application step of applying a first application liquid to an application target; and a second application step of applying and layering a second application liquid onto the first application liquid applied in the first application step.
(2) The method for producing a cellular tissue according to (1), wherein the second application step includes subjecting an interface between the first application liquid and the second application liquid to gelation while the first application liquid is kept fluid.
(3) The method for producing a cellular tissue according to (1) or (2), wherein the first application liquid contains a cell(s) and a gelling initiator.
(4) The method for producing a cellular tissue according to any one of (1) to (3), wherein the second application liquid contains a gelatinizer.
(5) A method for producing a cellular tissue, including: a first application step of applying a first application liquid containing a cell(s) and a gelling initiator to an application target; and a second application step of applying and layering a second application liquid containing a gelatinizer onto the first application liquid applied in the first application step, and starting a gelation reaction at an interface between the first application liquid and the second application liquid.
(6) The method for producing a cellular tissue according to any one of (1) to (5), further including a step of immersing the application target, to which the first application liquid and the second application liquid have been applied, in a culture medium to culture the cell(s).
(7) The method for producing a cellular tissue according to any one of (1) to (6), wherein the first application liquid contains a thickening polysaccharide.
(8) The method for producing a cellular tissue according to any one of (1) to (7), wherein the second application liquid contains a thickening polysaccharide.
(9) The method for producing a cellular tissue according to any one of (3) to (8), wherein the gelling initiator is thrombin.
(10) The method for producing a cellular tissue according to any one of (4) to (9), wherein the gelatinizer is fibrinogen, collagen, gelatin, or a mixture of two or more thereof.
(11) The method for producing a cellular tissue according to any one of (7) to (10), wherein the thickening polysaccharide is sodium hyaluronate, sodium alginate, or a mixture thereof.
(12) The method for producing a cellular tissue according to any one of (3) to (11), wherein the cell(s) is a cardiomyocyte(s).

In addition, as more specific embodiments of the second aspect in embodiment 3 according to the invention, for example, the following is provided.
(13) A cellular tissue formation set including at least a first container containing a first application liquid containing a cell(s) and a gelling initiator and a second container containing a second application liquid containing a gelatinizer.
(14) The cellular tissue formation set according to (13), wherein the first application liquid contains a thickening polysaccharide.
(15) The cellular tissue formation set according to (13) or (14), wherein the second application liquid contains a thickening polysaccharide.
(16) The cellular tissue formation set according to any one of (13) to (15), wherein the gelling initiator is thrombin.
(17) The cellular tissue formation set according to any one of (13) to (16), wherein the gelatinizer is fibrinogen, collagen, gelatin, or a mixture of two or more thereof.
(18) The cellular tissue formation set according to any one of (14) to (17), wherein the thickening polysaccharide is sodium hyaluronate, sodium alginate, or a mixture thereof.
(19) The cellular tissue formation set according to any one of (13) to (18), wherein the cell(s) is a cardiomyocyte(s).
(20) A cellular tissue produced by the method for producing a cellular tissue according to any one of (1) to (12).
(21) The method for producing a cellular tissue according to any one of (1) to (12), wherein the first application step is performed by contact application with an application needle.

### (Embodiment 4)

Hereinafter, embodiment 4, which relates to a method for producing a cellular tissue according to the invention and a cellular tissue prepared by the production method, will be described. Note that, in the description for embodiment 4, elements having substantially the same action, structure, and function as of the above embodiment 1, 2, or 3 may have the same reference numerals so as to omit and avoid redundant description.

In the method for producing a cellular tissue according to embodiment 4 of the invention, the application process described in detail in the above embodiments 1 and 3 is used to apply a cell-containing application liquid (first application liquid) attached to the tip 9a of the application needle 9 onto an application target. It is configured such that after the first application liquid is applied, the second application liquid is applied and layered onto the first application liquid. The process of applying the second application liquid is not particularly limited, and may use, for example, manual application such as liquid droplet application by, for instance, a syringe or a dropper or a bioprinting technique such as an inkjet process or a dispenser process.

In the method for producing a cellular tissue of embodiment 4, the application process described in the above embodiment 3 may be used to form a liquid micro-droplet spot(s). A plurality of cell tissues can be produced in one well (bottomed well), which is small cultureware (see Fig. 16 (c)).

The method for producing a cellular tissue of embodiment 4 has the same steps as in the method for producing a cellular tissue of the above embodiment 3. That is, in the method for producing a cellular tissue of embodiment 4, as shown in the schematic diagram of Fig. 18, the first application liquid (first liquid) is applied to the application target by using the application needle 9. In the first application step of applying the first application liquid, an application operation is repeated a desired number of times. Next, a second application liquid (second liquid) containing a gelatinizer is applied so as to cover the first application liquid (first liquid) applied in the first application step. A culture medium, as a third application liquid (third liquid), is added after a contact surface between the first application liquid and the second application liquid and the outer peripheral region thereof are subject to gelation. The inside of the first liquid, namely the first application liquid is kept in a liquid state while the fluidity is maintained to give a dome structure. Thus, the cells are settled and precipitated over time, and aggregated, deposited, and layered in a lower portion of the first application liquid. This results in production of a high-density three-dimensional cellular tissue. The lower right panel in Fig. 18 schematically illustrates a state in which cells are precipitated in the gel dome.

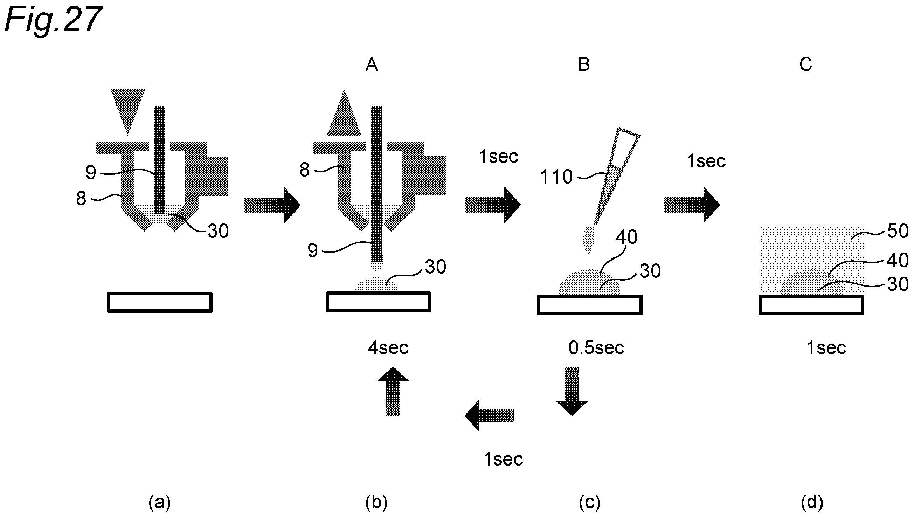

Fig. 27 is a schematic diagram illustrating each step in the method for producing a cellular tissue of embodiment 4. (a) of Fig. 27 illustrates a state in which the application needle 9 is dipped in the first application liquid (first liquid) 30 charged in the application liquid container 8 (application needle dipping process). (b) of Fig. 27 illustrates a first application step A of applying the first application liquid 30 by using the application needle 9. In the first application step A, the first application liquid 30 is applied onto the application target a desired number of times. (c) of Fig. 27 illustrates the second application step B in which the second application liquid 40 is applied so as to cover the first application liquid 30 applied in the first application step A. In embodiment 4, the second application step B is executed by an application operation using a micropipette 110. Next, the third application liquid 50 is applied (added) and a culture medium is provided such that the entire second application liquid 40 is immersed (in-medium formation step C). (d) of Fig. 27 illustrates a state in which the second application liquid 40 layered onto the first application liquid 30 on the substrate is immersed in the culture medium (50) by the in-medium formation step C.

As described above, the method for producing a cellular tissue of embodiment 4 like in the method for producing a cellular tissue of the above embodiment 3 has a first application step A of applying a first application liquid 30 containing a cell(s) and a gelling initiator to an application target; and a second application step B of applying and layering a second application liquid 40 containing a gelatinizer so as to cover the first application liquid 30 applied in the first application step A, and starting a gelation reaction. Thereafter, a culture medium is provided so that the second application liquid 40 covering the first application liquid 30 is immersed therein (in-medium formation step C).

The method for producing a cellular tissue of embodiment 4 may be used to efficiently produce a cellular tissue, for instance, at each of a plurality of application target positions on the substrate. The plurality of application target positions may be respective positions in a plurality of wells of the cultureware, or a plurality of application target positions in one well may be provided to produce a plurality of cellular tissues on the one well (see (c) of Fig. 16).

In the method for producing a cellular tissue of embodiment 4, the first application step A shown in (b) of Fig. 27 is executed with respect to the first application target positions, which are the plurality of application target positions, and then the second application step B shown in (c) of Fig. 27 is executed. Next, the first application step A is performed on another second application target position while the position is shifted, and then the second application step B is performed. In this manner, the first application step A and the second application step B are sequentially executed for each of the plurality of application target positions. After the first application step A and the second application step B are performed on all the application target positions, the in-medium formation step C is performed on each application target position. In this in-medium formation step C, a culture medium is provided to each well (cultureware).

In the first application step A and the second application step B in the method for producing a cellular tissue of embodiment 4, the application process described above in example 1 was used. The micro-applicator 100 was used for the application operation of the first application liquid 30 during the first application step A. The application operation of the second application liquid 40 during the second application step B was performed by manually dropping the liquid using a micropipette.

### (1) The first application liquid (first liquid) 30 is as follows.

| | |
|---|---|
| iPS cell-derived cardiomyocytes: | Human cardiac fibroblasts = 3 : 1 |
| Cell count: | 8 x 10⁷ cells/mL |
| Sodium hyaluronate: | 13 mg/mL |
| Thrombin: | 800 unit/mL |
| PBS | |
| Viscosity: | 1.5 × 10⁴ mPa·s) |
| Application diameter: | About 1.0 mm (contact application by using an application needle) |

### (2) Second Application Liquid (Second Liquid) 40

| | |
|---|---|
| Amount added: | 15 µL |
| Sodium hyaluronate: | 0.5 mg/mL |
| Fibrinogen: | 10 mg/mL |
| PBS | |
| Viscosity: | 1 × 10³ mPa·s |

Manual application (addition) by using a micropipette

After the first application liquid 30 was added, the second application liquid was added within 5 sec.

### (3) Culture Medium (Third Liquid) 50

After completion of the applications onto a plurality of application target positions by the first application step A and the second application step B, the culture medium was manually added to the entire cultureware by using a micropipette.

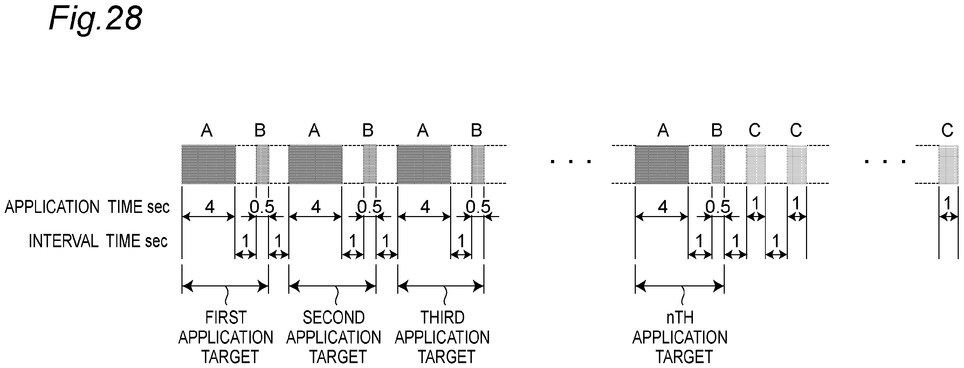

Fig. 28 is a time chart showing an example of specific application times of the first application step A, the second application step B, and the in-medium formation step C in the method for producing a cellular tissue of embodiment 4 and an interval time between the steps. As shown in Fig. 28, the first application step A is performed for 4 sec and the second application step B is performed for 0.5 sec on one application target position. In the first application step A, the application operation is repeated 10 times. In the second application step B, the second application liquid 40 is manually added with a micropipette.

As shown in the time chart of Fig. 28, first, the first application step A and the second application step B are executed on each application target position, and the in-medium formation step C is then executed on each application target position (for 1 sec). The interval time between the steps in which the first application step A, the second application step B, and the in-medium formation step C are performed is 1 sec. The application time and the interval time are changed, if appropriate, depending on the application conditions such as the composition contents of the first application liquid 30 and the second application liquid, the application amount, and the number of cellular tissues to be produced.

In the method for producing a cellular tissue of embodiment 4, as shown in the schematic diagram of Fig. 18 in the above embodiment 3, cells are suspended in the first application liquid 30, and the resulting first application liquid 30 is charged in the application liquid container 8. After the first application liquid 30 is charged in the application liquid container 8, the first application liquid 30 containing the cells is applied to the application target by using the application needle 9. Next, the second application liquid 40 is added so as to cover the first application liquid 30. As a result, gelation occurs from a contact surface between the first application liquid 30 and the second application liquid 40 to the outer periphery. After such gelation, a culture medium is added.

As shown in the lower panel of Fig. 18, the inside of the application liquids in the culture medium has a dome structure while the first application liquid 30 remains as a liquid. Thus, cells are precipitated inside the first application liquid 30 over time. As a result, a high-density three-dimensional cellular tissue was successfully produced.

In addition, the contact surface between fibrinogen and thrombin, which surface is the outer peripheral portion of the cellular tissue immediately after application, is subject to gelation and the inside thereof is kept in a liquid state. This configuration can prevent the cellular tissue from being dried.

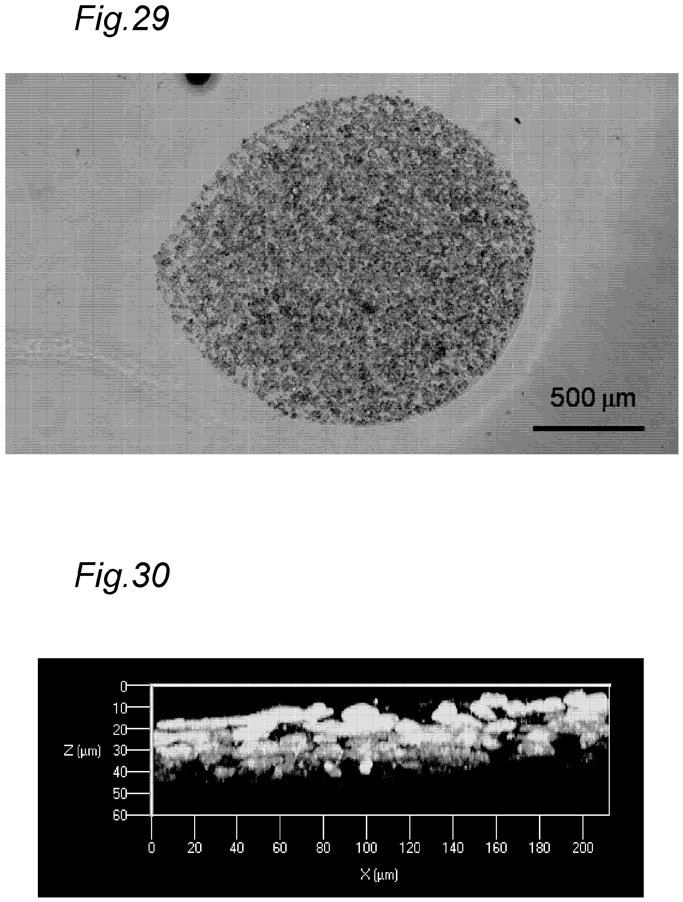

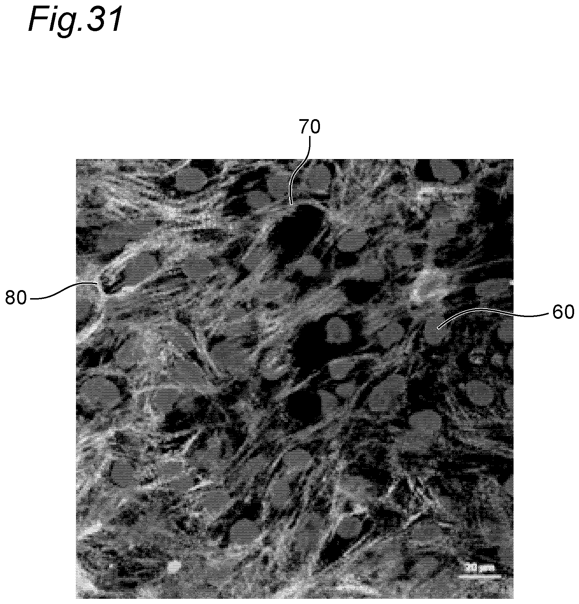

Fig. 29 is a photograph showing an image of a three-dimensional cellular tissue in the case of dispersing cardiomyocytes and thrombin in a first application solution 30. The state shown in Fig. 29 is a state before the cells are precipitated, and it can be seen from the state shown in Fig. 29 that there is a gap between the cells. As a result of measuring the thickness of the cellular tissue shown in Fig. 29, it was found that the cellular tissue had a thickness of about 50 µm and was layered at high density. Fig. 30 is a photograph showing an image of the result of measuring the thickness of the produced cellular tissue by fluorescent color. Further, the results of staining of cell nuclei, actin, and troponin demonstrated that the cells were alive, and verified expression of actin and troponin characteristic of cardiomyocytes. Fig. 31 is a photograph showing an image of the results of observing the produced cellular tissue by fluorescent staining. In Fig. 31, a substantially round shape is a cell nucleus 60, a dark linear-shaped protein is actin 70, and a whitish linear-shaped protein is troponin 80.

Note that adhesion between cells is promoted by coating the membrane surface of cells suspended in the first coating solution 30 with a protein, which is a cell adhesion factor. Also, a cell layering process or cell accumulation process may be used to construct a three-dimensional structure with high cell density.

### [Application Operation on Multi-Well Cultureware]

As described above, the first application step A, the second application step B, and the in-medium formation step C are performed for each well of multi-well cultureware having a plurality of wells. This can prevent cell death due to drying and enables a plurality of cellular tissues to be produced at a high speed with high precision and high shape stability. That is, while the cell death is suppressed, the waiting time for the first application liquid 30 applied at the initial stage of the first application step A is reduced. This makes it possible to decrease the frequency of switching the application mechanism for the application operation of each application liquid and the frequency of moving the stage on which the cultureware is placed. Thus, the time required for the whole steps can be prevented from becoming longer. The method for producing a cellular tissue of embodiment 4 is so executed to be able to produce a plurality of three-dimensional cellular tissues in multi-well cultureware.

Note that the first application liquid 30, in which a gelling initiator and cells are mixed, and the second application liquid 40, which is a gelatinizer, may be mixed to produce a cellular tissue embedded in a gel. In this case, the gelation time should be from about 10 sec to about several min. This gelation time and the waiting time until the application (addition) of the third application liquid for in-medium formation may be integrated into the same step. This can not only reduce the cell death and the required time, but also produce a cellular tissue embedded in a stable shaped gel at a given application position.

In the case of producing a cellular tissue in multi-well cultureware such as a 96-well plate, the application operation is executed according to the time chart shown in Fig. 28. During this application operation, a waiting time occurs between the application of the second application liquid 40 and the application of the third application liquid (culture medium) 50. However, fibrinogen, which covers the outer periphery in the vicinity of the contact surface between fibrinogen and thrombin, is subject to gelation. Also, the inside of the first application liquid 30 has thrombin as a liquid. Thus, during this waiting time, the cells are precipitated and the cells are then layered. This can create a high-density three-dimensional cellular tissue. Further, a culture medium as the third application liquid may be applied to culture the three-dimensional cellular tissue for a long period of time without causing cell death.

The number of repetitions of the application operation of the first application liquid 30 (first application step A) and the application operation of the second application liquid 40 (second application step B), that is, the number of repetitions of the first application step A and the second application step B until the third application liquid 50 is applied (in-medium formation step C) may be set to, for instance, 4 times, 6 times, 8 times, 12 times, 24 times, 36 times, 48 times, 96 times, or 384 times. The number of repetitions may be set according to various conditions such as the application amounts of the first application liquid 30 and the second application liquid 40 and the gelation time of gelatinizer such as fibrinogen and thrombin.

Note that in the time chart shown in Fig. 28, the configuration has been described in which the application operation of the first application liquid 30 is performed multiple times in the first application step A and the application operation of the second application liquid 40 is performed once in the second application step B. However, the invention is not limited to such a configuration, and the number of application operations in each application step may be set, if appropriate. For example, the application operation in one first application step A and one second application step B may be alternately performed to execute continuous application on multiple application target positions. In each application step, the first application liquid 30 or the second application liquid 40 may be applied a plurality of times for application operations. In the case of such multiple application operations, the application volume of the application liquid on the application target position increases. In particular, by carrying out the application operation multiple times for the first application liquid 30 having suspended cells, the number of cells inside the applied first application liquid 30 increases and the cell layer thickness also increases.

Note that in the method for producing a cellular tissue of embodiment 4, sodium hyaluronate is mixed, as a thickener, in the first application liquid 30 and the second application liquid 40. However, another thickening polysaccharide such as sodium alginate may be used. The thickener is effective in improving the shape retainability after the application liquid is applied. That is, the thickener has an effect of suppressing the shape deformation caused by the application liquid discharge after the application. In particular, sodium hyaluronate has high adhesiveness, adheres well to cultureware and cells, and is thus suitable for improving the shape retainability in the application liquid. Further, sodium hyaluronate is of biological origin and also has an advantage of high compatibility with cell growth.

In the method for producing a cellular tissue of embodiment 4, as the first application liquid (containing a gelling initiator) 30 and the second application liquid (containing a gelatinizer) 40, it is possible to use, in addition to a combination of thrombin and fibrinogen, for instance, calcium chloride and sodium alginate, calcium chloride and carrageenan, an alcohol compound and tamarind seed gum.

In addition, in the method for producing a cellular tissue of embodiment 4, iPS cell-derived cardiomyocytes and human cardiac fibroblasts are exemplified. However, normal human fibroblasts, human vascular endothelial cells, HePG2 cells, or cells obtained by mixing two or more thereof may be used. The use also makes it possible to produce, at a high speed, a cellular tissue with high precision and high shape stability while cell death due to drying is prevented.

Note that in the method for producing a cellular tissue of embodiment 4, the application operation of the first application liquid 30 is performed by contact application using the application needle 9. Because of this, there is no concern about nozzle clogging, which may occur when a cellular tissue is produced using an inkjet device or a dispenser device. This configuration can correspond to a further broad range of liquid and allows for micro-application. In addition, the applications of the second application liquid 40 and the third application liquid (culture medium) 50 are executed using an inkjet device or a dispenser device. The inkjet device permits micro-application, and has excellent high-speed performance. The dispenser device can correspond to a wider range of liquids than inkjet and is suitable for relatively large volumes of application.

Meanwhile, in the method for producing a cellular tissue of embodiment 4, an example is shown in which the first application liquid 30 contains cells and a gelling initiator and the second application liquid 40 contains a gelatinizer. However, regarding the combination, the first application liquid 30 may contain cells (excluding cardiomyocytes) and a gelatinizer and the second application liquid 40 may contain a gelling initiator. In this case, a gel in which cells are embedded is formed in a short time. This makes it possible to produce a cellular tissue that has high shape stability and an intended shape, and, in particular, is aligned on a flat surface of interest.

### [Production of Multiple Cellular Tissues on One Well (Cultureware)]

A plurality of cellular tissues may be produced by executing, on one well (cultureware), the application operation of the first application liquid 30 (first application step A), the application operation of the second application liquid 40 (second application step B), and the application operation of the third application liquid 50 (in-medium formation step C) in the method for producing a cellular tissue of embodiment 4. Each step in the method for producing a cellular tissue of embodiment 4 may be performed at multiple positions in each well (cultureware). This makes it possible to produce a plurality of independent cellular tissues on one well (cultureware).

For instance, cellular tissues may be produced on a 96-well plate. In this case, cells spread over the entire culture surface of each well (cultureware) in a 96-well plate by a cell sub-culture procedure using a conventional micropipette. The diameter may be 6.5 mm (the diameter of the well) (see (a) of Fig. 16). However, use of the method for producing a cellular tissue in embodiment 4 enables the first application liquid 30 and the second application liquid 40 to be applied with the diameter of 1.0 mm or less. This makes it possible to produce very small cellular tissues inside one well (see (b) of Fig. 16).

Further, the first application step A using the first application liquid 30 and the second application step B using the second application liquid 40 are performed at each of multiple positions on the culture surface of each well (cultureware) in a 96-well plate. This allows for production of cellular tissues present independently at multiple spots on the same well (cultureware) (see (c) of Fig. 16).

In order to produce independent cellular tissues at multiple spots on the same well (cultureware), the diameter of the tip 9a of the application needle 9 was set to 1.0 mm, so that each cellular tissue having a diameter of about 1.0 mm was successfully produced. In addition, by changing the diameter of the tip 9a of the application needle 9, cellular tissues having different diameters can be produced. For example, cellular tissues were produced using the application needle 9 having a tip 9a diameter of 330 µm. In this case, 6 independent cellular tissues with a diameter of about 330 µm were produced in the same cultureware (see Fig. 12).

As described above, cell tissues may be independently produced at multiple spots on one well (cultureware). This makes it possible to efficiently conduct drug efficacy and pharmacological evaluation during drug discovery process and drug assessment during safety evaluation. In conventional method for producing a cellular tissue, only one cellular tissue was produced on one well (cultureware). Due to this, just one analysis result was obtained from one well (cultureware). However, the method for producing a cellular tissue of embodiment 4 of the invention allows for production of a plurality of cellular tissues on one well (cultureware). This can provide cellular tissues such that multiple analysis results can be obtained from one well (cultureware) and the evaluation efficiency is high due to high-throughput performance.

As described above, according to the method for producing a cellular tissue of embodiment 4, the first application liquid 30 obtained by mixing a gelling initiator and cells and the second application liquid 40 as a gelatinizer were applied in sequence to each of a plurality of wells (bottomed wells: cultureware) in multi-well cultureware. Finally, the third application liquid 50 as a culture medium was applied to each well. This made it possible to prevent cell death due to drying and enabled three-dimensional cellular tissues to be produced on multi-well cultureware at a high speed with high precision and high shape stability.

Further, the micro-applicator may be used to apply the first application liquid 30 obtained by mixing a gelatinizer and cells to multiple spots on a flat culture surface of each well (cultureware). This can reduce the total number of cells while producing many independent cellular tissues with less variation in size in a simple step.

### (Embodiment 5)

Hereinafter, embodiment 5, which relates to a method for producing a cellular tissue according to the invention and a cellular tissue prepared by the production method, will be described with reference to the accompanying drawings. Note that, in the description for embodiment 5, elements having substantially the same action, structure, and function as of the above embodiment 1, 2, 3, or 4 may have the same reference numerals so as to omit and avoid redundant description.

In the method for producing a cellular tissue according to embodiment 5 of the invention, the application process described in detail in the above embodiment 1 is used to apply a cell-containing first application liquid 30 attached to the tip 9a of the application needle 9 onto an application target. It is configured such that after the first application liquid 30 is applied, the second application liquid 40 is applied and layered onto the first application liquid 30. The application process using the second application liquid 40 is not particularly limited, and may use, for example, manual application such as liquid droplet application by, for instance, a syringe or a dropper or a bioprinting technique such as an inkjet process or a dispenser process.

The method for producing a cellular tissue of embodiment 5 differs from those of the above embodiments 1, 2, 3, and 4. The difference point is that a series of application steps is used to produce, on one cultureware, each cellular tissue containing different types of cells.

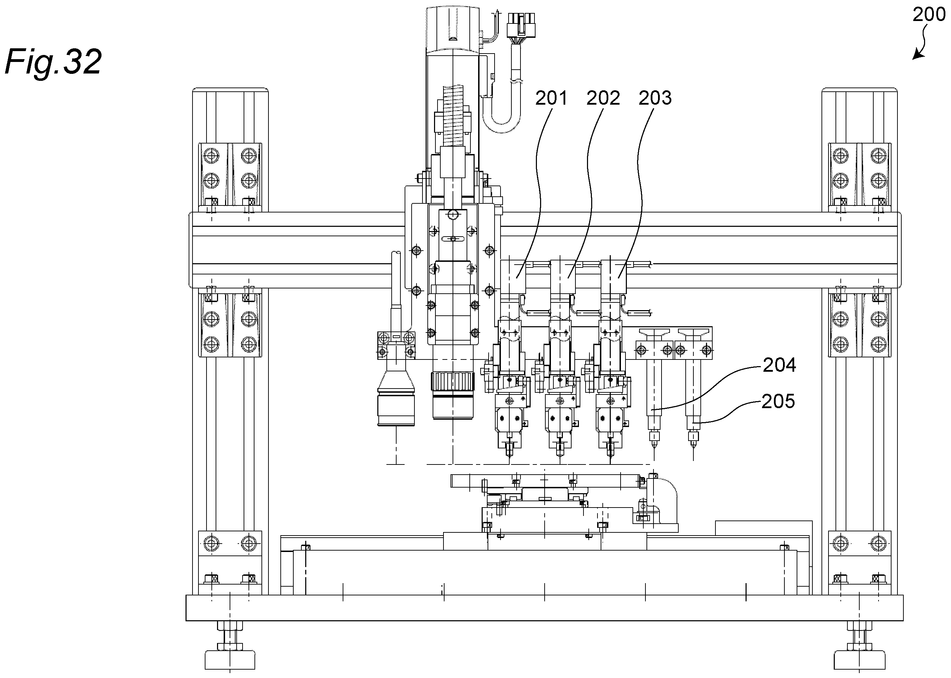

Fig. 32 is a front view showing a micro-applicator 200 used in the method for producing a cellular tissue of embodiment 5. The micro-applicator 200 is provided with three application units 201, 202, and 203. Each has an application liquid container filled with a solution containing a different type of cells (first application liquid 30X, 30Y, or 30Z). The configuration of these application units 201, 202, and 203 is similar to that of the application unit 6 in the micro-applicator 1 described in embodiment 1. The application needle 9 is likewise used to conduct contact application on an application target in this configuration.

In addition, the micro-applicator 200 is provided with two dispensers 204 and 205. The two dispensers 204 and 205 are arranged side by side with the three application units 201, 202, and 203. The first dispenser 204 is configured to apply and layer the second application liquid 40 onto the applied first application liquid 30 (30X, 30Y, or 30Z). The second dispenser 205 is configured to apply and provide a culture medium such that the second application liquid 40 layered on the applied first application liquid 30 (30X, 30Y, or 30Z) is immersed.

The micro-applicator 200 in embodiment 5 is configured to apply three different first application liquids 30 (30X, 30Y, and 30Z) having different types of cells (X cells, Y cells, and Z cells) by the three application units 201, 202, and 203, respectively. That is, in the micro-applicator 200, the first application unit 201 executes the first application step using the first application liquid 30X obtained by mixing a gelling initiator and cells X; the second application unit 202 executes the first application step using the first application liquid 30Y obtained by mixing a gelling initiator and cells Y; and the third application unit 203 executes the first application step using the first application liquid 30Z obtained by mixing a gelling initiator and cells Z.

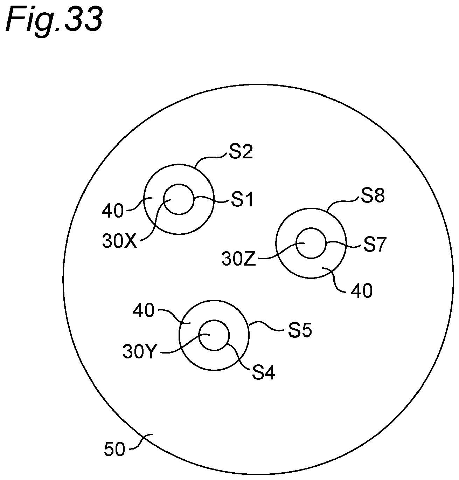

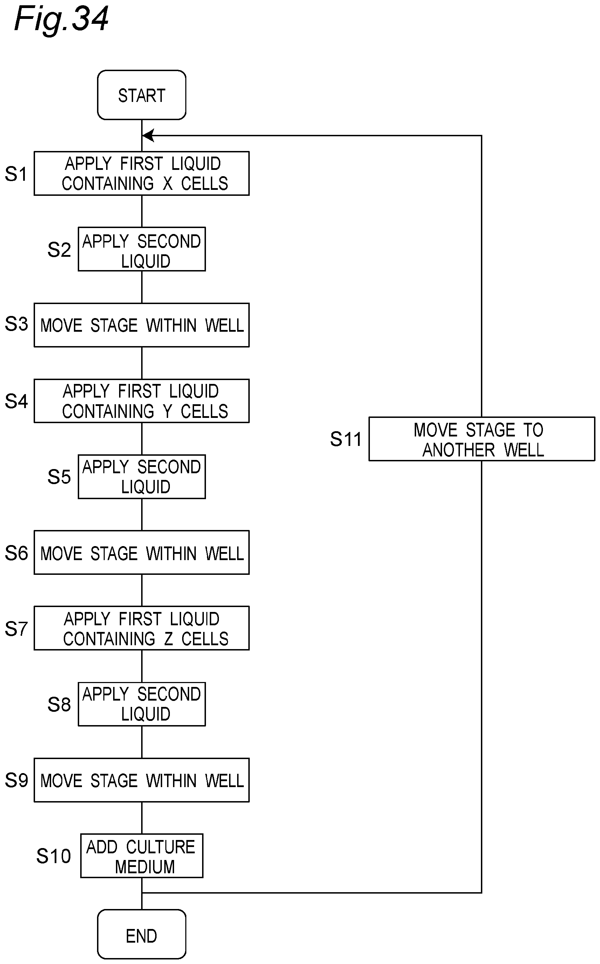

Fig. 33 is a plan view schematically illustrating an example in which independent cellular tissues were produced at three spots in one well (cultureware) by using the micro-applicator 200. Fig. 34 is a flowchart for producing the three types of cellular tissues shown in Fig. 33. After multi-well cultureware is fixed at a predetermined position in the micro-applicator 200, the cellular tissue production operation in embodiment 5 is started.

When the cellular tissue production operation in embodiment 5 is started, a designated first position on a specified well in the multi-well cultureware is set to be the application target position of the cells X by the first application unit 201. The first application step is executed by the first application unit 201 set, and the first application liquid 30X obtained by mixing the gelling initiator and the cells X as the first liquid is applied to the first position (step 1). In step 2, the second application step is executed such that the second application liquid 40, which is a gelatinizer as the second liquid, is applied and layered onto the first application liquid 30X by the first dispenser 204. After the second application liquid 40 is applied, the stage is moved to the second position in the specified well, which position is the application target position of the cells Y (step 3).

The first application step is executed at the second position as the application target position by the second application unit 202, and the first application liquid 30Y obtained by mixing the gelling initiator and the cells Y as the first liquid is applied to the second position (step 4). In step 5, the second application liquid 40, which is a gelatinizer as the second liquid, is applied and layered onto the first application liquid 30Y by the first dispenser 204 (second application step). After the second application liquid 40 is applied, the stage is moved to the third position in the specified well, which position is the application target position of the cells Z (step 6).

The first application step is executed at the third position as the application target position by the third application unit 203, and the first application liquid 30Z obtained by mixing the gelling initiator and the cells Z as the first liquid is applied to the third position (step 7). In step 8, the second application step is executed such that the second application liquid 40, which is a gelatinizer as the second liquid, is layered onto the first application liquid 30Z by the first dispenser 204. After the second application liquid 40 is applied, the stage is moved so as to set the inside of the specified well to be an application target (step 9). Then, an in-medium formation step is executed to apply (add) the third application liquid 50 as a culture medium to the well by the second dispenser 205.

The first application step, the second application step, and the in-medium formation step are executed on the well specified in the multi-well cultureware as described above. Subsequently, the first application step, the second application step, and the in-medium formation step are executed on the next well. In this way, the first application step, the second application step, and the in-medium formation step are sequentially executed on the corresponding well in the multi-well cultureware. As a result, different types of cellular tissues are produced in each well.

Note that in embodiment 5, the case where three types of cell tissue are produced on each well (identical cultureware well) has been described. However, the invention is not limited to such a production method. For instance, the identical well may have a plurality of the same type of cellular tissues and a different type of cellular tissue. Alternatively, at least two types of different cellular tissues may be produced. As such, various combinations are included.

In addition, specific examples of the combination in the case of applying different types of cells to one well include:
(1) a combination of X cells: iPS cell-derived cardiomyocytes, Y cells: iPS cell-derived neurons, and Z cells: primary hepatocytes;
(2) a combination of X cells: iPS cell-derived cardiomyocytes, Y cells: iPS cell-derived neurons, and Z cells: HepG2;
(3) a combination of X cells: iPS cell-derived cardiomyocytes, Y cells: iPS cell-derived neurons, and Z cells: HeLa cells; or
(4) a combination of X cells: iPS cell-derived cardiomyocytes, Y cells: human cardiac fibroblasts, Z cells: human cardiovascular endothelial cells.

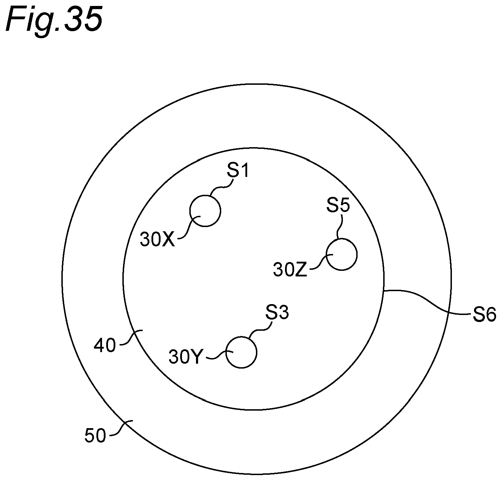

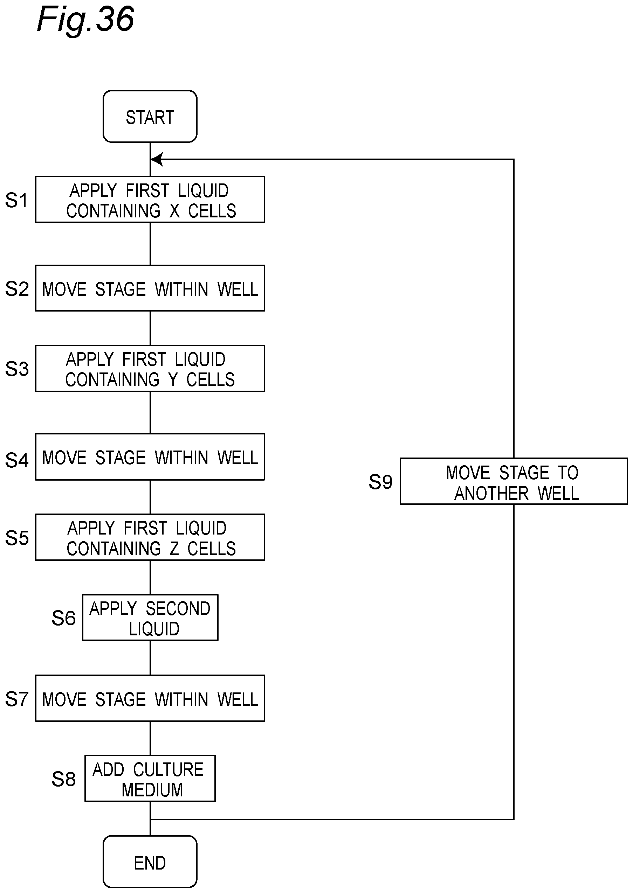

Fig. 35 is a plan view schematically illustrating an example in which independent cellular tissues are produced at three spots in one well (cultureware), and is a modification example of producing the cellular tissues shown in Fig. 33 described above. Fig. 36 is a flowchart for producing the three types of cellular tissues shown in Fig. 35.

In the method for producing a cellular tissue shown in Figs. 35 and 36, the cellular tissue production operation is started after multi-well cultureware is fixed at a predetermined position. When the cellular tissue production operation is started, a designated first position on a specified well in the multi-well cultureware is set to be the application target position of the cells X applied by the first application unit 201. The first application step is executed by the first application unit 201 set, and the first application liquid 30X obtained by mixing the gelling initiator and the cells X as the first liquid is applied to the first position (step 1).

After the first application liquid 30X is applied, the stage is moved to the second position in the specified well as the application target position of the cells Y (step 2). The first application step is executed at the second position as the application target position by the second application unit 202, and a first application liquid 30Y obtained by mixing the gelling initiator and the cells Y as the first liquid is applied to the second position (step 3).

After the first application liquid 30Y is applied, the stage is moved to the third position in the specified well as the application target position of the cells Z (step 4). The first application step is executed at the third position as the application target position by the third application unit 203, and a first application liquid 30Z obtained by mixing the gelling initiator and the cells Z as the first liquid is applied to the third position (step 5).

In step 6, the second application step is executed such that the second application liquid 40, which is a gelatinizer as the second liquid, is layered onto the first application liquids 30X, 30Y, and 30Z by the first dispenser 204. After the second application liquid 40 is applied, the stage is moved so as to set the inside of the specified well to be an application target (step 7). Then, an in-medium formation step is executed to apply (add) the third application liquid 50 as a culture medium to the well by the second dispenser 205.

As described above, in the method for producing a cellular tissue shown in Fig. 36, the first application step is sequentially performed on the well in the multi-well cultureware. Then, the second application step and the in-medium formation step are executed in this configuration. As such, the method for producing a cellular tissue is performed on the multi-well cultureware, and different types of cellular tissue are produced in each well.

Note that in embodiment 5, the micro-applicator 200 is configured to produce three types of cell tissue. However, the invention is not limited to such a configuration. Application units may be provided depending on the type of cells to be produced. This allows for production of a plurality of types of cellular tissues.

As described above, the micro-applicator 200 according to embodiment 5 of the invention is so configured to be able to produce a plurality of types of cellular tissues in one well (cultureware). Such production of a plurality of types of cellular tissues in one well (cultureware) has following advantages.
(1) For instance, there may be a drug acting differently on cells X or cells Y. At the same time, a cellular tissue containing the cells X and a cellular tissue containing the cells Y may be produced in one well. In this case, when the drug is applied to the well, the efficacy on each cell type (X or Y) can be checked in the well at the same time. For example, an anthracycline-based anticancer agent is a drug that often causes cardiac toxicity. By allowing cancer cells and iPS cell-derived cardiomyocytes to coexist in one well and adding the anticancer agent thereto, it is possible to simultaneously observe the intrinsic effect of the anticancer agent on the cancer cells and the cardiotoxic effect on the iPS cell-derived cardiomyocytes.
(2) Since different types of cellular tissues share a culture medium, it is possible to share a cytokine produced from some cells or a metabolite of a drug added. For example, whether or not a drug metabolized in cells X acts on cells Y can be observed.

The micro-applicator 200 in embodiment 5 has been used to describe a method for producing a plurality of types of cell tissues on each well in multi-well cultureware. However, the micro-applicator 200 may be used to produce a plurality of cellular tissues containing the same type of cells on each well in the multi-well cultureware. In this way, by producing a plurality of cellular tissues on one well, a plurality of samples can be evaluated on one well. In addition, a plurality of samples can be imaged by one imaging during analysis of an image or a video. This can shorten the imaging time and can thus shorten analysis time. For instance, in the case of using 96-well cultureware, only one sample can be produced in one well by a conventional method for producing a cellular tissue. However, the method for producing a cellular tissue of embodiment 5 makes it possible to produce 4 cellular tissue samples c with a diameter of about 1.0 mm on one well. Thus, the method for producing a cellular tissue of embodiment 5 makes is possible to produce 384 (96 × 4) cellular tissue samples by using 96-well w cultureware. In addition, for instance, 20 sec of imaging may be required for video analysis of one well. This case needs 80 sec for video imaging of 4 samples because in a conventional method for producing a cellular tissue, one well has one sample. By contrast, in the method for producing a cellular tissue of embodiment 5, 4 samples are produced inside one well, so that the video imaging is greatly shortened to 20 sec.

As described above, according to the method for producing a cellular tissue of embodiment 5, the first application liquid 30 obtained by mixing a gelling initiator and cells and the second application liquid 40 as a gelatinizer were applied in sequence to each of a plurality of wells (bottomed wells: cultureware) in multi-well cultureware. Finally, the third application liquid 50 as a culture medium was applied to each well. This can prevent cell death due to drying and enables three-dimensional cellular tissues to be produced on multi-well cultureware at a high speed with high precision and high shape stability.

Further, the micro-applicator may be used to apply the first application liquid 30 obtained by mixing a gelatinizer and cells to multiple spots on a flat culture surface of each well (cultureware). This can reduce the total number of cells while producing many independent cellular tissues with less variation in size in a simple step.

The invention has been described with reference to embodiments in some detail. However, this configuration is exemplary, and the details of the disclosure of the embodiments may have a change. The elements in the embodiments of the invention may be replaced by other elements and the combinations and the order thereof may be changed, which can be realized without departing from the scope and the spirit of the invention claimed.

The method for producing a cellular tissue of the invention makes it possible to produce various cellular tissues in large quantity in a highly reliable manner. Thus, the invention is a critical technology in drug discovery research and regenerative medicine study, and is therefore highly industrially applicable.

## Claims

1. A method for producing a cellular tissue, comprising:
a first application step of applying a first application liquid to an application target; and
a second application step of applying and layering a second application liquid onto the first application liquid applied in the first application step to subject an interface between the first application liquid and the second application liquid to gelation.

2. The method for producing a cellular tissue according to claim 1, wherein the second application step comprises subjecting an interface between the first application liquid and the second application liquid to gelation while the first application liquid is kept fluid.

3. The method for producing a cellular tissue according to claim 1 or 2, wherein the first application liquid comprises a cell(s) and a gelling initiator.

4. The method for producing a cellular tissue according to any one of claims 1 to 3, wherein the second application liquid comprises a gelatinizer.

5. A method for producing a cellular tissue, comprising:
a first application step of applying a first application liquid containing a cell(s) and a gelling initiator to an application target; and
a second application step of applying and layering a second application liquid containing a gelatinizer onto the first application liquid applied in the first application step, and starting a gelation reaction at an interface between the first application liquid and the second application liquid.

6. A method for producing a cellular tissue, comprising:
a first application step of applying a first application liquid containing a cell(s) to an application target; and
a second application step of applying and layering a second application liquid containing collagen, gelatin and/or agar as a gelatinizer onto the first application liquid applied in the first application step, and starting a gelation reaction by heating or cooling at an interface between the first application liquid and the second application liquid.

7. A method for producing a cellular tissue, comprising:
a first application step of applying a first application liquid containing a cell(s) to an application target; and
a second application step of applying and layering a second application liquid containing a photocrosslinkable gelatin hydrogelatinizer onto the first application liquid applied in the first application step, and starting a gelation reaction by light irradiation at an interface between the first application liquid and the second application liquid.

8. The method for producing a cellular tissue according to any one of claims 1 to 7, further comprising a step of immersing the application target to which the first application liquid and the second application liquid have been applied, in a culture medium to culture the cell(s).

9. The method for producing a cellular tissue according to any one of claims 1 to 8, wherein the first application liquid comprises a thickening polysaccharide.

10. The method for producing a cellular tissue according to any one of claims 1 to 9, wherein the second application liquid comprises a thickening polysaccharide.

11. The method for producing a cellular tissue according to claim 3 or 5, wherein the gelling initiator is thrombin.

12. The method for producing a cellular tissue according to claim 4 or 5, wherein the gelatinizer is fibrinogen, collagen, gelatin, or a mixture of two or more thereof.

13. The method for producing a cellular tissue according to claim 9 or 10, wherein the thickening polysaccharide is sodium hyaluronate, sodium alginate, or a mixture thereof.

14. The method for producing a cellular tissue according to any one of claims 3 to 13, wherein the cell(s) is a cardiomyocyte(s).

15. A cellular tissue formation set comprising at least a first container containing a first application liquid containing a cell(s) and a gelling initiator and a second container containing a second application liquid containing a gelatinizer.

16. The cellular tissue formation set according to claim 15, wherein the first application liquid comprises a thickening polysaccharide.

17. The cellular tissue formation set according to claim 15 or 16, wherein the second application liquid comprises a thickening polysaccharide.

18. The cellular tissue formation set according to any one of claims 15 to 17, wherein the gelling initiator is thrombin.

19. The cellular tissue formation set according to any one of claims 15 to 18, wherein the gelatinizer is fibrinogen, collagen, gelatin, or a mixture of two or more thereof.

20. The cellular tissue formation set according to any one of claims 16 to 19, wherein the thickening polysaccharide is sodium hyaluronate, sodium alginate, or a mixture thereof.

21. The cellular tissue formation set according to any one of claims 15 to 20, wherein the cell(s) is a cardiomyocyte(s).

22. A cellular tissue produced by the method for producing a cellular tissue according to any one of claims 1 to 14.

23. The method for producing a cellular tissue according to any one of claims 1 to 14, wherein the first application step is performed by contact application with an application needle.

24. The method for producing a cellular tissue according to any one of claims 1 to 14 or 23, wherein application operations of the first and second application steps are executed at a plurality of application target positions on an identical well and an in-medium formation step is executed.

25. The method for producing a cellular tissue according to claim 24, wherein the application operations of the first and second application steps are executed at the plurality of application target positions on the identical well, and the in-medium formation step is then executed.

26. The method for producing a cellular tissue according to claim 24, wherein the application operations of the first and second application steps are executed at the plurality of application target positions on the identical well, and then the in-medium formation step is executed at the application target positions.

27. The method for producing a cellular tissue according to claim 24, wherein the application operation of the first step is executed at the plurality of application target positions on the identical well, and then the application operation of the second application step is executed at the plurality of application target positions on the identical well, and then the in-medium formation step is executed at the plurality of application target positions on the identical well.

28. The method for producing a cellular tissue according to any one of claims 24 to 27, wherein the in-medium formation step comprises applying a third application liquid so that the second application liquid including the first application liquid is immersed in the third application liquid.

29. The method for producing a cellular tissue according to any one of claims 24 to 28, wherein a cellular tissue containing a same type of cell(s) is produced at the plurality of application target positions in the identical well.

30. The method for producing a cellular tissue according to any one of claims 24 to 28, wherein a cellular tissue containing a different type of cell(s) is produced at the plurality of application target positions in the identical well.

31. The method for producing a cellular tissue according to any one of claims 24 to 30, wherein an inkjet technique or a dispenser technique is used for the second application step of applying and layering the second application liquid onto the first application liquid.

32. The method for producing a cellular tissue according to any one of claims 24 to 31, wherein a two-component gelling solution is used as the application liquids during cellular tissue formation.

33. Cultureware comprising a plurality of cellular tissues in an identical well.

34. The cultureware according to claim 33, wherein the cultureware comprises a plurality of same type of cellular tissues in the identical well.

35. The cultureware according to claim 33, wherein the cultureware comprises a plurality of different types of cellular tissues in the identical well.

36. The cultureware according to any one of claims 33 to 35, wherein the cultureware comprises a plurality of wells, each well comprising a plurality of cellular tissues.
